(19) Europäisches Patentamt
European Patent Office
Office européen des brevets

(11) **EP 4 291 251 B1**

(12) **EUROPEAN PATENT SPECIFICATION**

(45) Date of publication and mention
of the grant of the patent:
**19.02.2025 Bulletin 2025/08**

(21) Application number: **22705782.5**

(22) Date of filing: **15.02.2022**

(51) International Patent Classification (IPC):
*A61K 51/08* (2006.01)   *A61P 35/00* (2006.01)
*C07K 14/31* (2006.01)   *A61K 103/10* (2006.01)
*A61K 103/30* (2006.01)

(52) Cooperative Patent Classification (CPC):
**A61P 35/00; A61K 51/08; C07K 14/31;**
**C07K 2319/20**

(86) International application number:
**PCT/EP2022/053621**

(87) International publication number:
**WO 2022/171892 (18.08.2022 Gazette 2022/33)**

(54) **HER2-BINDING POLYPEPTIDE**

HER2-BINDENDES POLYPEPTID

POLYPEPTIDE SE LIANT À HER2

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **15.02.2021 EP 21157213**

(43) Date of publication of application:
**20.12.2023 Bulletin 2023/51**

(73) Proprietor: **Affibody AB**
**171 65 Solna (SE)**

(72) Inventors:
• **FREJD, Fredrik**
**112 20 Stockholm (SE)**
• **TOLMACHEV, Vladimir**
**752 66 Uppsala (SE)**

(74) Representative: **AWA Sweden AB**
**Box 45086**
**104 30 Stockholm (SE)**

(56) References cited:
**WO-A1-2009/080810   WO-A1-2015/028550**
**CN-A- 110 251 695**

• **LIU YONGSHENG ET AL: "Comparative**
**Preclinical Evaluation of HER2-Targeting ABD-**
**Fused Affibody® Molecules 177Lu-ABY-271 and**
**177Lu-ABY-027: Impact of DOTA Position on**
**ABD Domain", PHARMACEUTICS, vol. 13, no. 6,**
**7 June 2021 (2021-06-07), pages 839,**
**XP055918171, DOI: 10.3390/**
**pharmaceutics13060839**
• **XU TIANQI ET AL: "Drug Conjugates Based on a**
**Monovalent Affibody Targeting Vector Can**
**Efficiently Eradicate HER2 Positive Human**
**Tumors in an Experimental Mouse Model",**
**CANCERS, vol. 13, no. 1, 30 December 2020**
**(2020-12-30), pages 85, XP055918177, DOI:**
**10.3390/cancers13010085**
• **FELDWISCH J ET AL: "Design of an Optimized**
**Scaffold for Affibody Molecules", JOURNAL OF**
**MOLECULAR BIOLOGY, ACADEMIC PRESS,**
**UNITED KINGDOM, vol. 398, no. 2, 30 April 2010**
**(2010-04-30), pages 232 - 247, XP027000361,**
**ISSN: 0022-2836, [retrieved on 20100310], DOI:**
**10.1016/J.JMB.2010.03.002**

- **S. AHLGREN ET AL: "Targeting of HER2-Expressing Tumors with a Site-Specifically 99mTc-Labeled Recombinant Affibody Molecule, ZHER2:2395, with C-Terminally Engineered Cysteine", THE JOURNAL OF NUCLEAR MEDICINE, vol. 50, no. 5, 16 April 2009 (2009-04-16), US, pages 781 - 789, XP055282969, ISSN: 0161-5505, DOI: 10.2967/jnumed.108.056929**
- **WÅLLBERG HELENA ET AL: "Molecular Design and Optimization of 99m Tc-Labeled Recombinant Affibody Molecules Improves Their Biodistribution and Imaging Properties", THE JOURNAL OF NUCLEAR MEDICINE, vol. 52, no. 3, 14 February 2011 (2011-02-14), US, pages 461 - 469, XP055829570, ISSN: 0161-5505, DOI: 10.2967/jnumed.110.083592**
- **OROUJENI MARYAM ET AL: "Preclinical Evaluation of 99mTc-ZHER2:41071, a Second-Generation Affibody-Based HER2-Visualizing Imaging Probe with a Low Renal Uptake", INTERNATIONAL JOURNAL OF MOLECULAR SCIENCES, vol. 22, no. 5, 9 March 2021 (2021-03-09), pages 2770, XP055829572, DOI: 10.3390/ijms22052770**

Remarks:
  The complete document including Reference Table(s) and the Sequence Listing(s) can be downloaded from the EPO website

**Description**

Field of the invention

The present disclosure concerns new polypeptides which bind to Human Epidermal Growth Factor Receptor 2 (in the following referred to as HER2). The present disclosure also relates to use of such HER2-binding polypeptides as a diagnostic agent, prognostic agent and/or medicament, more particularly use thereof in the diagnosis, prognosis and/or treatment of forms of cancer characterized by over-expression of HER2.

Background

HER2 is a transmembrane receptor belonging to the receptor tyrosine kinase superfamily. HER2 is overexpressed in a substantial fraction of breast, gastroesophageal and ovarian cancers, and its elevated expression is associated with poor prognosis. Tumors with high HER2 expression respond to specific targeting therapeutics, so information concerning a patient's HER2 expression level is critical for disease management. Currently practiced methods to determine HER2 expression status include the analysis of biopsy material, which is invasive and complicated, requiring multiple samplings and making it difficult to elucidate heterogeneous expression of HER2 or changes in HER2 expression in response to therapy.

Radionuclide molecular imaging, on the other hand, promises non-invasive detection of HER2 in both primary tumors and metastases, without false negative results originating from sampling errors. The first clinical studies proving this approach were performed with the monoclonal antibody trastuzumab, labelled with the single photon emitter indium-111. Further progress in the use of antibody-based probes was achieved by the use of a long-lived positron emitter, zirconium-89, and application of positron emission tomography (PET), which improved sensitivity and resolution. Still, imaging probes based on therapeutic antibodies clear slowly from blood. This causes a low imaging contrast (even 4-5 days after injection) and an elevated dose burden. Using smaller imaging probes, e.g. probes based on engineered scaffold proteins, enables the reduction of the optimal imaging time to a few hours after injection and increases the contrast of imaging, in turn enhancing sensitivity.

One scaffold protein useful for generating HER2-binding molecules, e.g. for the purpose of radionuclide imaging, is the protein Z derivative of domain B of staphylococcal Protein A. Z variants are based on a 58 amino acid, cysteine-free scaffold, and can be synthesized chemically or produced in bacteria by recombinant DNA technology. Z variants are further characterized by their small size and high thermal stability, as well as the possibility of site-specific radiolabeling and potentially very high target affinity. Different generations of HER2-binding Z variants are disclosed in WO2005/003156, WO2009/080810 and WO2015/028550.

The first generation of HER2-binding Z variants (see WO2005/003156) demonstrated an impressive capability to image HER2-expressing tumors in preclinical (Orlova et al (2006), Cancer Res 66:4339-4348; Ahlgren et al (2009) J Nucl Med 50:781-789) and clinical (Baum et al(2010), J Nucl Med 51(6):892-897) studies. In a second generation, the Z variant scaffold was extensively re-engineered, with 11 of 45 amino acids in the non-binding part substituted (WO2009/080810, Feldwisch et al(2010) J Mol Biol 398:232-247). These substitutions improved the yield of peptide synthesis of Z variant molecules and their storage stability. Residual binding to IgG and IgM was essentially eliminated to facilitate blood clearance, and the hydrophilicity of water-exposed surface was enhanced by substitution of alanines by serines.

HER2-binding Z variants of this second generation have been used in PET imaging in the form of a DOTA-conjugated, $^{68}$Ga-labelled radiotracer molecule. It has demonstrated an excellent sensitivity and specificity in the detection of HER2 expressing metastases in patients with breast cancer (Sörensen et al (2016), Theranostics 6(2):262-271). A typical clinical investigation using this tracer would give an effective dose in the range of 5-6 mSv, which is appreciably lower compared to doses typical for PET imaging with antibody reagents (14-22 mSv). While PET imaging is relatively available in Northern America and Western Europe, it is less accessible in South America, Asia, Africa, and some European countries. Here, use of SPECT is more common. The same imaging molecule has been tested for SPECT too, using $^{111}$In as the label (Sörensen et al (2014), J Nucl Med 55(5):730-735), but this nuclide is expensive and requires medium energy collimators, which decrease resolution and sensitivity.

The use of $^{99m}$Tc (T1/2 = 6 h) is much more attractive from a clinical point of view. This nuclide is cheap and readily available because of its generator production. The decay scheme of $^{99m}$Tc makes it almost ideal for radiopharmaceutical labeling because of its optimal half-life, benign dosimetry and possibility to use low-energy high-resolution collimators, which increases the sensitivity of imaging. Labeling of first generation Z variants with $^{99m}$Tc was well investigated, in particular using peptide-based chelators. It was shown that the composition of such chelators and their position in the Z variant molecules has the potential to substantially modify the biodistribution of the $^{99m}$Tc-labeled molecules and their excretion pathway (Hofström et al (2013), J Med Chem 56:4966-4974). Furthermore, it was shown that placing a cysteine residue at the C-terminus of a Z variant sequence (thus creating the C-terminal sequence -KVDC; SEQ ID NO:4) forms an $N_3S$ chelator useful for stable coupling of $^{99m}$Tc (Ahlgren et al (2009), J Nucl Med 50:781-789). The $^{99m}$Tc-labelled HER2-binding molecule $Z_{HER2:2395}$ of Ahlgren *et al* (2009, *supra*) demonstrated improved targeting specificity and high contrast upon imaging of HER2 expression (tumor-to-blood ratio 121 $\pm$ 24, 4 h after injection) in a murine model. However, $Z_{HER2:2395}$ had a high renal uptake (140-190 %ID/g, 4 h after injection), which is undesirable for imaging.

$^{188}$Re has also been tested in this context. It is a high energy, beta-emitting radioisotope with a short half-life (16.98 h), which allows generation of radiopharmaceuticals for diagnostics and therapy of malignant tumors. The short half-life makes it safer for patients, staff and environment. Radiation from $^{188}$Re includes both beta and gamma components. The high beta emission (784 keV) ensures a high therapeutic effect and the low-abundant gamma emission (155 keV) enables operators to obtain visual information in conventional gamma cameras. Furthermore, a rhenium generator can easily be provided at a clinical site.

It has been found that both the amino acid composition and the order of amino acid residues in peptide-based chelators influence biodistribution and targeting properties (Wållberg et al(2011), J Nucl Med 52:461-469; Altai et al (2012), Amino Acids 5:1975-1985). The best imaging contrast yet was demonstrated by the first generation HER2-binding Z variant $^{99m}$Tc-$Z_{HER2:V2}$, containing a C-terminal -GGGC chelating sequence (SEQ ID NO:5). It has been shown that the [$^{99m}$Tc](Tc=O)-GGGC complex acts as an non-residualizing label. Activity which was reabsorbed and rapidly internalized in the kidney was rapidly excreted. In tumors, where internalization of Z variant molecules is slow, the activity was retained for a long time due to the high affinity of the Z variant for HER2 on cellular membranes (Wållberg *et al (2011), supra)*. This provided a combination of a good imaging construct with low renal uptake of $^{99m}$TC-$Z_{HER2:V2}$.

Based on these results, the -GGGC chelator could be seen as a candidate for labeling also of the second generation of HER2-binding Z variants. However, rearranging amino acids in the scaffold sequence risk creating an additional chelating pocket by clustering amino acids with electron-donating side-chains. It has been suggested that such side-chains could participate in the complexing of technetium (Cyr et al (2007), J Labelled Comp Radiopharm 50(suppl 1):S9), leading to loss of labeling site-specificity and to the modification of stability of a conjugate and its pharmacokinetics. For example, introduction of an HEHEHE-tag (SEQ ID NO:6) at the N-terminus of $Z_{HER2:V2}$ resulted in a noticeable change in the biodistribution of the resulting construct, and particularly in much higher renal uptake (Lindberg et al (2012), Tumour Biol 33(3):641-651). It has also been shown that even making a minor change to $^{99m}$Tc-$Z_{HER2:V2}$, introducing an N-terminal purification tag, has a detrimental effect on the labeling, in this case in a clinical setting (Cai et al (2020), Iran J Radiol e96419, published online on 20 January 2020). Furthermore, the binding site composition of another Z variant, the EGFR-binding molecule $Z_{EGFR:2377}$, contains a chelating pocket which competes with the -GGGC chelator and makes its use impossible (Oroujeni et al (2018), Amino Acids 50:981-994).

In conclusion, it is far from given that a re-engineered HER2-specific Z variant is compatible with stable labeling with $^{99m}$Tc using a -GGGC chelating sequence in its scaffold.

Disclosure of the invention

It is an object of the present disclosure to satisfy the requirements of a continued provision of agents with an affinity for HER2, through the provision of a polypeptide that is characterized by specific binding to HER2.

A related object of the disclosure is a HER2-binding polypeptide which exhibits little or no non-specific binding.

It is another object of the disclosure to provide a HER2-binding polypeptide that can readily be used as a moiety in a fusion polypeptide.

Another object is the provision of a HER2-binding polypeptide, which solves one or more of the known problems experienced with existing antibody reagents.

A further object of the disclosure is to provide a HER2-binding polypeptide, which is amenable to use in therapeutic applications.

A further object of the disclosure is to provide a HER2-binding polypeptide, which is amenable to use in diagnostic applications.

A further object of the disclosure is to provide a HER2-binding polypeptide, which is amenable to use in prognostic applications.

A related object is to find new forms for the treatment, inhibition and/or targeting, in the clinical setting, of cancer diseases characterized by an overexpression of HER2 protein.

It is another object of the disclosure to provide a HER2-binding polypeptide which can be produced using recombinant protein expression or synthesized by chemical peptide synthesis.

A related object is to provide a HER2-binding polypeptide which can be labeled with a radionuclide as produced, without any additional step of conjugating a chelating group to the polypeptide.

Another object is to find a HER2-binding polypeptide that exhibits an improved stability vis-à-vis known HER2-binding agents, optionally leading also to a prolonged shelf-life upon storage.

Yet another object of the disclosure is to achieve a HER2-binding polypeptide that exhibits a low antigenicity and/or immunogenicity when used *in vivo* in a mammal.

It is another object of the disclosure to provide a HER2-binding polypeptide with a beneficial biodistribution upon administration to a mammal.

These and other objects are met by the different aspects of the present disclosure. Thus, in a first aspect, the disclosure provides a HER2-binding polypeptide, whose amino acid sequence comprises AEAKYAKEMR NAYWEIALLP NLTNQQKRAF IRKLYDDPSQ SSELLSEAKK
LSESQGGGC (SEQ ID NO:1)

In accordance herewith, the present inventors have found that a HER2-binding polypeptide comprising SEQ ID NO:1 exhibits surprising advantages, for example in comparison with the previously disclosed HER2-binding polypeptides $Z_{HER2:V2}$ in Wållberg *et al* (2011, *supra),* denoted H2BPP2 in the following and having SEQ ID NO:2, and $Z_{HER2:2395}$ in Ahlgren *et al* (2009, *supra),* denoted H2BPP3 in the following and having SEQ ID NO:3. Non-limiting examples of such advantages, which each individually may be exhibited by one or more embodiments of the polypeptide according to this aspect of the disclosure, are as follows:

- The disclosed polypeptide comprises fewer amino acid residues that could cause problems, such as low yield and success rate, in chemical synthesis of the polypeptide sequence, such as asparagine and aspartic acid.
- The disclosed polypeptide comprises fewer amino acid residues that confer surface hydrophobicity, and thus has a more hydrophilic profile than previously described, related HER2-binding polypeptides. This implies fewer problems with low solubility and aggregation. Without wishing to be bound by theory, it is also currently believed that the more hydrophilic characteristics act to shift the biodistribution of the polypeptide upon administration to a host, from a hepatobiliary pathway (excretion through the liver) towards a more desired renal pathway (excretion through the kidneys).
- The disclosed polypeptide comprises fewer amino acid residues that are associated with stability problems of polypeptides, such as asparagine, aspartic acid and the dipeptide asparagine-proline. Asparagine is susceptible to deamidation, aspartic acid is susceptible to isomerization and the asparagine-proline bond is susceptible to cleavage, and they therefore contribute to the non-homogeneity of the final product.
- The disclosed polypeptide lacks amino acid residues that, in a similar sequence context, have been found to increase the interaction with immunoglobulins containing a heavy chain variable domain from VH3 (Silverman G.J., Int. Rev. Immunol. 1992;9(1):57-78). Without wishing to be bound by theory, it is currently believed that the replacement of such amino acid residues in the polypeptides according to the invention reduces the antigenicity of the polypeptide upon administration of same to a host.
- The disclosed polypeptide comprises fewer potential T-cell epitopes than previously known HER2-binding Z variants, as shown in Example 4 below, and is therefore likely to be less immunogenic.

A polypeptide according to this aspect of the disclosure can be used as an alternative to HER2-binding antibodies and to known HER2-binding Z variants in diverse applications. As non-limiting examples, it is useful in the treatment of cancers characterized by HER2 overexpression, in inhibiting cell signaling by binding to the HER2 on a cell surface, in the diagnosis and/or prognosis of cancers characterized by HER2 overexpression both *in vivo* and *in vitro,* in targeting of other therapeutic, diagnostic or prognostic agents to cells overexpressing HER2, in histochemical methods for the detection of HER2, in methods of separation and other applications. The polypeptide according to this aspect may prove useful in any method which relies on affinity for HER2 of a reagent. Thus, the polypeptide may be used as a detection reagent, a capture reagent or a separation reagent in such methods, as a diagnostic and/or prognostic agent for diagnostics and/or prognostics *in vivo* or *in vitro,* or as a therapeutic agent in its own right or as a means for targeting other therapeutic or diagnostic agents to the HER2 protein. Methods that employ the polypeptide according to this aspect of the disclosure *in vitro* may be performed in different formats, such as in microtiter plates, in protein arrays, on biosensor surfaces, on tissue sections, and so on.

Different modifications of, and/or additions to, the polypeptide according to this aspect of the disclosure may be performed in order to tailor the polypeptide to the specific use intended, without departing from the scope of the present teaching. Such modifications and additions are described in more detail below, and may comprise additional amino acids comprised in the same polypeptide chain, or labels and/or therapeutic agents that are chemically conjugated or otherwise bound to the polypeptide according to this aspect of the disclosure.

The terms "HER2-binding" and "binding affinity for HER2" as used in this specification refer to a property of a polypeptide which may be tested for example by ELISA or by the use of surface plasmon resonance (SPR) technology.

For example, as described in the examples below, HER2-binding affinity may be tested in an experiment in which HER2, or a fragment thereof, is immobilized on a sensor chip of a surface plasmon resonance (SPR) instrument, and the sample containing the polypeptide to be tested is passed over the chip. Alternatively, the polypeptide to be tested is immobilized on a sensor chip of the instrument, and a sample containing HER2, or a fragment thereof, is passed over the chip. The skilled person may then interpret the results obtained by such experiments to establish at least a qualitative measure of the binding affinity of the polypeptide for HER2. If a quantitative measure is desired, for example to determine a $K_D$ value for the interaction, surface plasmon resonance methods may also be used. Binding values may for example be defined using a Biacore T200 instrument (Cytiva) or a ProteOn XPR 36 (Bio-Rad) instrument. HER2 is suitably immobilized on a sensor chip of the instrument, and samples of the polypeptide whose affinity is to be determined are prepared by serial dilution and injected in random order. $K_D$ values may then be calculated from the results using for example the 1:1 Langmuir binding model of the BIAevaluation 4.1 software, or other suitable software, provided by the instrument manufacturer.

This aspect of the disclosure also encompasses polypeptides in which the HER2-binding polypeptide described above is present as a HER2-binding domain, to which additional amino acid residues have been added at either terminal, in particular at the N terminus. These additional amino acid residues may play a role in the binding of HER2 by the polypeptide, but may also serve other purposes, related for example to one or more of the production, purification, stabilization, coupling or detection of the polypeptide. Such additional amino acid residues may comprise one or more amino acid residues added for purposes of chemical coupling. Such additional amino acid residues may also comprise a "tag" for purification or detection of the polypeptide, such as a hexahistidyl ($H_6$) tag, or a "myc" tag or a "FLAG" tag. Such a tag may for example enable interaction with antibodies specific to the tag or immobilized metal affinity chromatography (IMAC) as the case may be. The skilled person is aware of other alternatives.

The "additional amino acid residues" discussed above may also constitute one or more polypeptide domain(s) with any desired function, such as the same binding function as the first, HER2-binding domain, or another binding function, or an enzymatic function, or a fluorescent function, or mixtures thereof.

Thus, the disclosure encompasses multimers of the polypeptide comprising SEQ ID NO:1. It may be of interest, e.g. when using the polypeptide according to the invention for diagnosis or treatment of cancer or in a method of purification of HER2, to obtain even stronger binding of HER2 than is possible with one polypeptide according to the invention. In this case, the provision of a multimer, such as a dimer, trimer or tetramer, of the polypeptide may provide the necessary avidity effects. The multimer may consist of a suitable number of polypeptides according to the invention. The linked polypeptide "units" in a multimer according to the invention may be connected by covalent coupling using known organic chemistry methods, or expressed as one or more fusion polypeptides in a system for recombinant expression of polypeptides, or joined in any other fashion, either directly or via a linker, for example an amino acid linker.

Additionally, "heterogenic" fusion polypeptides, in which the polypeptide comprising SEQ ID NO:1 constitutes a first domain, or first moiety, and the second and further moieties have other functions than binding HER2, are also contemplated and fall within the ambit of the present disclosure. The second and further moiety/moieties of such a fusion polypeptide may comprise a binding domain with affinity for another target than HER2. Non-limiting examples of targets for such second and further moiety/moieties may be selected from the group consisting of CD3, CD137 (4-1BB), CTLA-4, EGFR, HER3, VEGF, PD1, PD-L1 and cMet. The result is then a fusion polypeptide having at least one HER2-binding domain and at least one domain with affinity for said other target molecule. This makes possible the creation of multispecific reagents that may be used in several applications, such as used as therapeutic and/or diagnostic and/or prognostic agents, or as reagents for capture, detection or separation. The preparation of such multispecific multimers of polypeptides, in which at least one polypeptide domain comprises SEQ ID NO:1, may be effected as described above for the multimer of several HER2-binding "units".

The second or further moiety or moieties may be a variant of a domain derived from protein A of *Staphylococcus aureus,* such as a Z variant, or comprise an unrelated, naturally occurring or recombinant, protein having a binding affinity for a target (or a fragment thereof retaining the binding capability of the naturally occurring or recombinant protein).

Another example of such a binding protein, which has an affinity for human serum albumin and may be used as fusion partner with the polypeptide according to the invention, is one of the albumin binding domains from protein G of Streptococcus strain G148 (Nygren P-Å et al (1988) Mol Recogn 1:69-74), such as the GA1, GA2 or GA3 domain. The GA3 domain of protein G is also denoted ABD, i.e. albumin binding domain. Derivatives of ABD with improved properties vis-à-vis the wild type GA3 domain of protein G have been disclosed, for example in WO2009/016043, WO2012/004384 and WO2014/048977. A fusion polypeptide between the HER2-binding polypeptide comprising SEQ ID NO:1 and an albumin binding domain of streptococcal protein G thus also falls within the scope of the present disclosure. When the disclosed polypeptide is administered to a human subject as a diagnostic, prognostic or therapeutic agent, or as a targeting agent, the fusion thereof to a moiety which binds serum albumin may prove beneficial, in that the half-life *in vivo* of such a fusion protein is likely to be extended compared with the half-life of the HER2-binding moiety in isolation (this principle has been described e.g. in WO91/01743).

Other possibilities for the creation of fusion polypeptides are also contemplated. Thus, the polypeptide according to the first aspect of the disclosure may be covalently coupled to a second or further moiety or moieties, which in addition to, or instead of, target binding exhibit other functions. One example is a fusion between one or more polypeptide(s) comprising SEQ ID NO:1 and an enzymatically active polypeptide serving as a reporter or effector moiety. Examples of reporter enzymes, which may be coupled to the polypeptide comprising SEQ ID NO:1 to form a fusion protein, are known to the skilled person and include enzymes such as β-galactosidase, alkaline phosphatase, horseradish peroxidase, carboxypeptidase. Other options for the second and further moiety or moieties of a fusion polypeptide according to the invention include fluorescent polypeptides, such as green fluorescent protein, red fluorescent protein, luciferase and variants thereof.

Other options for the second and further moiety or moieties of a fusion polypeptide according to the invention include a moiety or moieties for therapeutic applications. In therapeutic applications, other molecules may also be coupled, covalently or non-covalently, to the inventive polypeptide by other means. Non-limiting examples include enzymes for ADEPT (antibody-directed enzyme prodrug therapy) applications using the polypeptide according to the invention for direction of the effector enzyme (e.g. carboxypeptidase); proteins for recruitment of effector cells and other components of the immune system; cytokines, such as IL-2, IL-12, IFNγ, TNF, IP-10, GM-CSF; procoagulant factors, such as tissue factor, von Willebrand factor; toxins, such as ricin A, *Pseudomonas* exotoxin, calcheamicin, maytansinoid; toxic small molecules, such as auristatin analogs, doxorubicin.

With regard to the description above of fusion proteins incorporating the HER2-binding polypeptide according to the invention, it is to be noted that the designation of first, second and further moieties is made for clarity reasons to distinguish between the HER2-binding moiety or moieties on the one hand, and moieties exhibiting other functions on the other hand. These designations are not intended to refer to the actual order of the different domains in the polypeptide chain of the fusion protein. Thus, for example, said first moiety may without restriction appear at the N-terminal end, in the middle, or at the C-terminal end of the fusion protein.

In one embodiment of the first aspect of the disclosure, the HER2-binding polypeptide consists of SEQ ID NO:1.

The disclosure furthermore encompasses polypeptides in which the HER2-binding polypeptide described above has been provided with a label group, such as at least one fluorophore, biotin or a radioactive isotope, for example for purposes of detection of the polypeptide.

In particular, a second aspect of the disclosure relates to a radiolabeled polypeptide consisting of a radiochelate of a HER2-binding polypeptide according to the first aspect and a radionuclide.

A majority of radionuclides have a metallic nature. Because metals are typically incapable to form stable covalent bonds with elements presented in proteins and peptides, labeling of targeting proteins with radioactive metals is frequently performed with the use of chelators, i.e. multidentate ligands, which form non-covalent compounds, called chelates, with the metal. In the polypeptide according to the invention, the incorporation of a radionuclide is enabled through the provision of a chelating environment, through which the radionuclide may be coordinated, chelated or complexed to the polypeptide.

The polypeptide of the first aspect comprises a tetradentate chelating environment characterized as an $N_3S$ chelator. As the term $N_3S$ indicates, the four attaching groups of such a chelator are formed from three nitrogen atoms and one sulfur atom. In an $N_3S$ chelator, the N and S atoms are spatially arranged to provide a suitable "pocket" for complexing or attachment of the radioactive metal. In the polypeptide according to the disclosure, an $N_3S$ chelator is provided through the choice of -GGGC as the C-terminal amino acid residues in SEQ **ID** NO:1.

Thus, the polypeptide according to the first aspect, comprising a tetradentate chelating environment characterized as an $N_3S$ chelator, provided by the nitrogen atoms of three consecutive peptide bonds and a cysteine residue at the C-terminal of the polypeptide, may be used to provide a radiolabeled polypeptide according to the second aspect, consisting of a radiochelate of the HER2-binding polypeptide and a radionuclide.

In one embodiment, said radionuclide is suitable for medical imaging. In a more specific embodiment, the radionuclide is selected from the group consisting of $^{99m}Tc$, $^{51}Mn$, $^{52m}Mn$, $^{52}Mn$, $^{186}Re$ and $^{188}Re$. In a particular embodiment, the radionuclide suitable for medical imaging is **$^{99m}Tc.$**

In an alternative embodiment, said radionuclide is suitable for therapy. In a more specific embodiment, the radionuclide is selected from the group consisting of $^{188}Re$ and $^{188}Re$. In a particular embodiment, the radionuclide suitable for therapy is $^{188}Re$.

In these embodiments of the radiolabeled polypeptide according to the second aspect, the radionuclide is complexed with the HER2-binding polypeptide via the chelating environment provided by the C-terminal -GGGC sequence.

The present disclosure also encompasses compositions comprising a polypeptide according to the first aspect or a radiolabeled polypeptide according to the second aspect.

A third aspect of the disclosure provides a composition comprising the HER2-binding polypeptide of the first aspect, and at least one pharmaceutically acceptable excipient or carrier. Said composition may suitably be formulated for a purpose selected from storage, transport, lyophilization, concentration, or any combination thereof. In a particular embodiment, the composition according to this aspect is suitable for use as a medicament, a diagnostic agent in vivo or a prognostic agent in vivo without further manipulation. In another embodiment, said composition is suitable for lyophilization. Lyophilization of a composition according to this embodiment yields a lyophilized powder of said polypeptide. Said lyophilized powder of the polypeptide constitutes a further, fourth, aspect of the disclosure, and may offer advantages in terms of transport, storage, handling and radiolabeling of the polypeptide when it is intended for use as a medicament or as a diagnostic or prognostic agent *in vivo.*

A fifth aspect of the disclosure provides a composition comprising the radiolabeled polypeptide of the third aspect, and at least one pharmaceutically acceptable excipient or carrier. Suitably, said at least one pharmaceutically acceptable excipient or carrier is selected to enable or enhance the administration of radiolabeled polypeptide to a subject to be treated or imaged.

The person of skill in the art of pharmaceutical compositions and formulation is able to select the proper excipients or carriers for a given application of the HER2-binding polypeptide or radiolabeled polypeptide, to create a composition according to the third or fifth aspect without undue experimentation.

The present disclosure also encompasses different aspects of using the above-described HER2-binding polypeptide, whether radiolabeled or not, and compositions. It also encompasses various methods for treatment, diagnosis and prognosis in which the polypeptide is useful due to its binding characteristics. When referring to the "HER2-binding polypeptide" in the following description of these uses and methods, this term is intended to encompass the HER2-binding polypeptide alone, but also all those molecules based on this polypeptide described above that e.g. incorporate the HER2-binding polypeptide as a moiety in a fusion protein and/or are conjugated to a label, a chelator, a therapeutic and/or diagnostic agent and/or are provided with additional amino acid residues as a tag or for other purposes. As explained above, such fusion proteins, derivatives etc form a part of the present disclosure. The "HER2-binding polypeptide" is also intended to encompass the compositions and powders of the polypeptide or the radiolabeled polypeptide according to the third, fourth and fifth aspects above.

Thus, in a sixth aspect, the invention provides a method for *in vivo* imaging of the body of a mammalian, including human, subject having or suspected of having a cancer characterized by overexpression of HER2, the method comprising the steps:

- administration of a radiolabeled polypeptide according to the second aspect, comprising a radionuclide suitable for medical imaging, into the body of a mammalian subject; and
- obtaining an image of at least a part of the subject's body using a medical imaging instrument, said image indicating the presence of the radionuclide inside said body. In one embodiment, the image is obtained within 1-72 hours, such as within 1-24 hours, of administration of the radiolabeled polypeptide to the body. The time between the administration and the obtaining of the image is dependent on the half-life of the radionuclide used. It is possible to repeat the step of obtaining an image two, three or more times, whereby a series of images is obtained. This is useful when one seeks to follow the biodistribution over time of a targeting agent, and allows for pharmacokinetic studies. The skilled person appreciates that any number of images could be obtained, in order to achieve the requisite degree of time resolution in such a study.

In one embodiment of the imaging method according to this aspect, said administration is intravenous administration.

In one embodiment of the imaging method according to this aspect, the method comprises, before the administration step, a preparatory step of preparing a radiolabeled polypeptide according to the second aspect, which step comprises mixing of a polypeptide according to the first aspect with the radionuclide suitable for medical imaging.

In a more specific embodiment of the imaging method according to this aspect, the method comprises, before the administration step, a preparatory step of preparing a radiolabeled polypeptide of i) a polypeptide whose amino acid sequence comprises or consists of SEQ ID NO:1 with ii) $^{99m}$Tc, which step comprises mixing the polypeptide with $^{99m}$Tc-pertechnetate in the presence of appropriate reducing agent, e.g. stannous chloride, ascorbic acid or gentisic acid, in an appropriate buffer. This may also be performed in the presence of an intermediate weak chelator, e.g. tartrate, or citrate or gluconate.

If the polypeptide is present in the form of a lyophilized powder according to the fourth aspect, the preparatory step suitably also comprises a first step of reconstituting the powder in a suitable buffer for radiolabeling and administration.

In a related, seventh, aspect, the disclosure provides a method of diagnosing a cancer characterized by overexpression of HER2, comprising

- performing the method of *in vivo* imaging according to the sixth aspect, and
- establishing said diagnosis on the basis of the obtained images.

In another related, eighth, aspect, the disclosure provides a method of establishing a prognosis for a cancer characterized by overexpression of HER2, comprising

- performing the method of *in vivo* imaging according to the sixth aspect, and
- establishing said prognosis on the basis of the obtained images.

Furthermore, the disclosure provides, in a ninth aspect, a method of treatment of a mammalian, including human, subject having a cancer characterized by overexpression of HER2, comprising the step of administering a therapeutically effective amount of a radiolabeled polypeptide according to the second aspect, comprising a radionuclide suitable for therapy, to said subject.

In one embodiment of the treatment method according to this aspect, said administration is intravenous administration.

In one embodiment of the treatment method according to this aspect, the method comprises, before the administration step, a preparatory step of preparing a radiolabeled polypeptide according to the second aspect, which step comprises mixing of a polypeptide according to the first aspect with the radionuclide suitable for therapy.

In a more specific embodiment of the treatment method according to this aspect, said preparatory step comprises:

- mixing of a polypeptide according to the first aspect with perrhenate in appropriate buffer in the presence of appropriate reducing agent. This may also be performed in the presence of an intermediate weak chelator, e.g. tartrate, or citrate or gluconate; or
- mixing of a polypeptide according to the first aspect with a reactive intermediate of rhenium, such as a tricarbonyl complex.

In an even more specific embodiment of the treatment method according to this aspect, said preparatory step the method comprises, before the administration step, a preparatory step of preparing a radiolabeled polypeptide of i) a polypeptide whose amino acid sequence comprises or consists of SEQ ID NO:1 with ii) $^{188}$Re, which step comprises mixing the polypeptide with $^{188}$Re-perrhenate in the presence of appropriate reducing agent, e.g. stannous chloride, ascorbic acid or gentisic acid, in an appropriate buffer. This may also be performed in the presence of an intermediate weak chelator, e.g. tartrate, or citrate or gluconate.

If the polypeptide is present in the form of a lyophilized powder according to the fourth aspect, the preparatory step suitably also comprises a first step of reconstituting the powder in a suitable buffer for radiolabeling and administration.

In some embodiments of these diagnostic, prognostic and therapeutic aspects of the invention, said cancer is selected from breast cancer, ovarian cancer, gastric cancer, colorectal cancer, prostate cancer, bladder cancer, salivary cancer, lung cancer (in particular non-small cell lung cancer) and cancer in the esophagus (in particular gastric and gastroesophageal junction cancer). In a particular embodiment, said cancer is breast cancer.

The disclosure encompasses also the binding and targeting of HER2 positive metastases which originate from these cancer types but are localized in other parts of the subject's body. Particularly contemplated in this regard are HER2 positive metastases in the brain.

The disclosure furthermore provides the polypeptide according to the first aspect, the radiolabeled polypeptide according to the second aspect, the composition according to the third aspect, the lyophilized powder according to the fourth aspect, or the composition according to the fifth aspect, for use as a diagnostic agent *in vivo* or as a prognostic agent *in vivo* or as a medicament in any of the methods according to the sixth, seventh, eighth and ninth aspects.

A further, process-related, aspect of the disclosure is based on the surprising finding that it is possible to perform radiolabeling of the polypeptide as disclosed herein at a lower temperature than what has been previously considered practical or efficient. In this connection, it has been observed that the radiolabeling at such lower temperature generates fewer undesired byproducts of the reaction. The disclosure encompasses a method of radiolabeling of a polypeptide according to the first aspect, comprising a step of radiolabeling at a temperature in the interval 60-85 °C, for example at 65-80 °C, for example at 65-75 °C, in particular at approximately 70 °C, such as at 70 °C. The skilled person understands that the method of radiolabeling according to this aspect may be incorporated in the preparatory step of radiolabeling which is present in some embodiments of the imaging and treatment methods described above.

Another aspect of the disclosure relates to a nucleic acid molecule comprising a sequence which encodes a polypeptide according to the first aspect.

A further aspect of the disclosure relates to an expression vector comprising the nucleic acid molecule of the previous aspect, and other nucleic acid elements that enable production of the polypeptide through expression of the nucleic acid molecule.

Yet another aspect of the disclosure relates to a host cell comprising the expression vector of the previous aspect.

The latter three aspects of the invention are tools for the production of a polypeptide according to the invention, and the skilled person will be able to obtain them and put them into practical use without undue burden, given the information herein concerning the polypeptide that is to be expressed and given the current level of skill in the art of recombinant expression of proteins.

The polypeptide according to the disclosure may also be produced by other known means, including chemical synthesis or expression in different prokaryotic or eukaryotic hosts, including plants and transgenic animals.

The different aspects of the disclosure will now be illustrated in detail through the description of experiments conducted in accordance therewith. The examples to follow are not to be interpreted as limiting. In the examples, reference is made to the appended figures.

Brief description of the figures

Figure 1 is a listing of the amino acid sequences of a novel HER2-binding polypeptide containing a C-terminal GGGC chelator (H2BPP1; SEQ ID NO:1), a HER2-binding reference polypeptide containing a C-terminal GGGC chelator (H2BPP2; SEQ ID NO:2) and a HER2-binding reference polypeptide containing a C-terminal KVDC chelator (H2BPP3; SEQ ID NO:3).

Figure 2 shows circular dichroism spectra of (A) H2BPP1 and (B) H2BPP2 collected at 20 °C before (solid line) and after (broken line) variable temperature measurement (VTM).

Figure 3 shows RP-UPLC-MS total ion chromatograms (TIC) of (A) H2BPP1 and (C) H2BPP2 after heat treatment at 90 °C for 60 min (black lines). (B) and (D) are zoomed-in chromatograms of (A) and (C), respectively. Chromatograms of non-treated reference samples (grey lines) are included for comparison.

Figure 4 shows single cycle kinetic (SCK) SPR sensorgrams (black lines) demonstrating the binding of H2BPP1 to (A) human HER2 and (B) cynomolgus HER2 at concentrations of 0.3 and 3 nM, successively injected at the time points that are indicated by grey, vertical lines.

Figure 5 shows SCK SPR sensorgrams (black lines) demonstrating the absence of binding of H2BPP1 to human (A) HER1, (B) HER3 and (C) HER4 analyzed at the indicated concentrations, successively injected at the time points that are indicated by grey, vertical lines.

Figure 6 shows SDS-PAGE analysis of the $^{99m}$Tc-labeled polypeptides (1) $^{99m}$Tc-H2BPP1, (2) $^{99m}$Tc-H2BPP2 and (3) $^{99m}$Tc-H2BPP3, respectively; Signal (4) corresponds to $^{99m}$TcO$_4^-$. The signal was measured as digital light units (DLU) and is proportional to radioactivity at a given point of a lane in the SDS-PAGE gel.

Figure 7 shows in vitro binding specificity of (A, D) $^{99m}$Tc-H2BPP1, (B, E) $^{99m}$Tc-H2BPP2 and (C, F) $^{99m}$Tc-H2BPP3 to HER2-expressing SKOV-3 (A, B, C) and BT-474 (D, E, F) cell lines. For pre-saturation of HER2, a 500-fold molar excess of non-radiolabeled polypeptide was added. The data are presented as average (n=3) values $\pm$ SD.

Figure 8 shows normalized cellular retention of (A, D) $^{99m}$Tc-H2BPP1, (B, E) $^{99m}$Tc-H2BPP2 and (C, F) $^{99m}$Tc-H2BPP3 to HER2-expressing SKOV-3 (A, B, C) and BT-474 (D, E, F) cell-lines. The data are presented as an average (n=3) value $\pm$ SD. Where error bars are not seen, they are smaller than the respective point symbol.

Figure 9 shows uptake of the $^{99m}$Tc-labeled novel polypeptide H2BPP1 and the reference polypeptides H2BPP2 and H2BPP3, respectively, in different organs of female NMRI mice at 4 h after injection. 1 $\mu$g of labeled polypeptide (60 kBq, 100 $\mu$l in PBS) was injected into the tail vein. (A) Blood, (B) lung, (C) liver, (D) spleen, (E) stomach, (F) kidney, (G) muscle and (H) bone.

Figure 10 shows the in vivo specificity of $^{99m}$Tc-H2BPP1 in HER2-negative Ramos xenografts and HER2-positive SKOV-3 xenografts at 4 h after injection. The data are presented as an average (n=4) value $\pm$ SD.

Figure 11 shows the (A) biodistribution and (B) tumor-to-organ ratios of $^{99m}$Tc-H2BPP1 and $^{99m}$Tc-H2BPP2, as indicated, at 4 h after injection in BALB/C nu/nu mice bearing HER2-expressing SKOV-3 xenografts. The data are presented as the average (n=4) value $\pm$ SD.

Figure 12 shows imaging of HER2-positive SKOV-3 xenograft (right mouse) and HER2-negative Ramos xenograft (left mouse) in BALB/C nu/nu mice using $^{99m}$Tc-H2BPP1.

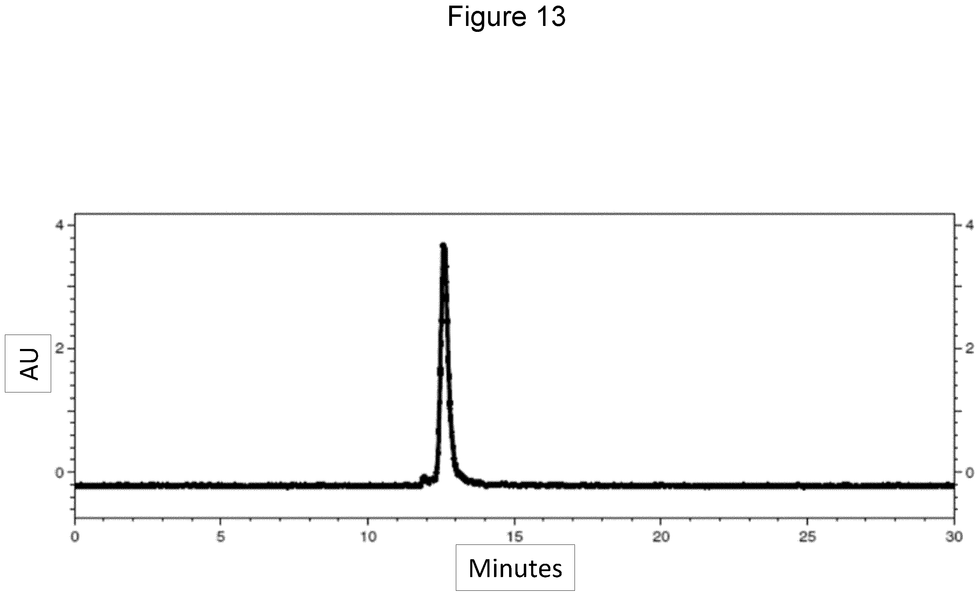

Figure 13 shows a chromatogram from RP-HPLC analysis of $^{188}$Re-H2BPP1 after labeling at 70 °C.

Figure 14 shows in vitro binding specificity of $^{188}$Re-H2BPP1 to HER2-expressing (A) SKOV-3 and (B) SK-BR-3 cell lines. For pre-saturation of HER2, a 400-fold molar excess of non-radiolabeled polypeptide was added to control dishes ("blocked"). The data are presented as average (n=3) values $\pm$ SD.

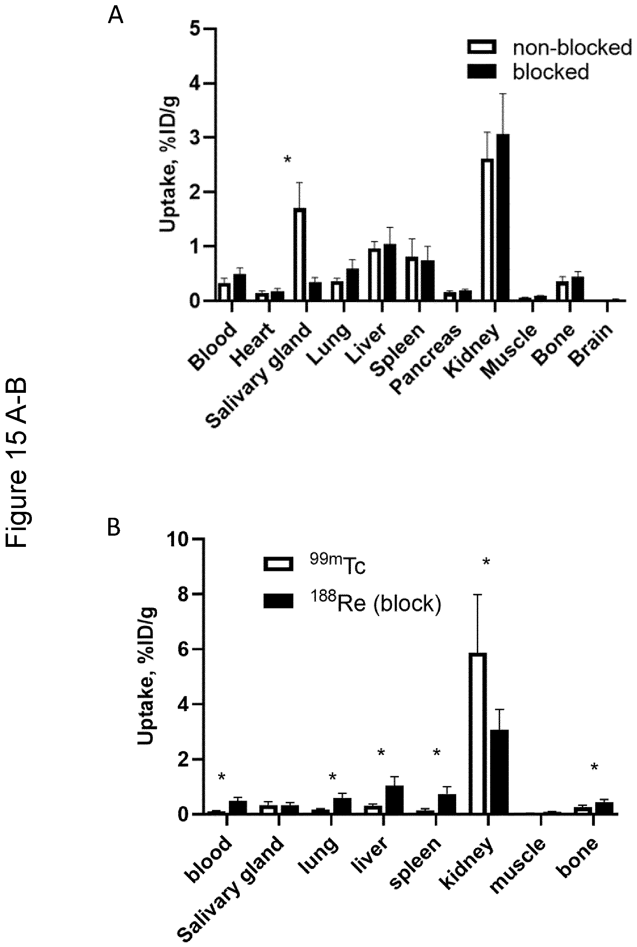

Figure 15 shows the biodistribution of imaging ligands in non-tumor bearing mice. (A) Results of saturation of Na/I symporter in salivary glands of NMRI mice. Supplementation of drinking water with NaI to one group of mice ("blocked") resulted in significant (p < 0.0013) reduction of activity uptake in salivary gland. The data are presented as average (n=4) values $\pm$ SD. (B) Comparison of biodistribution of $^{188}$Re-H2BPP1 and $^{99m}$Tc-H2BPP1 in NMRI mice, 4 h after injection. The data are presented as average (n=4) values $\pm$ SD. Asterisks mark significant (p<0.05 in unpaired t-test) difference.

Figure 16 shows imaging of HER2-positive SKOV-3 xenograft in BALB/C nu/nu mice 1 and 4 h after injection of $^{188}$Re-H2BPP1. Arrows point at tumors (T) and kidneys (K).

Figure 17 shows (A) comparison of $^{188}$Re-H2BPP1 in BALB/C nu/nu mice bearing HER2-expressing SKOV-3 and HER2-negative Ramos xenografts, with data presented as average (n=4) values $\pm$ SD; and (B) imaging of HER2-positive SKOV-3 xenograft (left mouse) and HER2-negative Ramos xenograft (right mouse) in BALB/C nu/nu mice

injected with $^{188}$Re-H2BPP1, arrows pointing at tumors (T).

Figure 18 shows growth of individual tumors in mice treated with (A) $^{188}$Re-H2BPP1 (3 times with 5 μg, 16 MBq, dissolved in 10% ethanol in water), (B) vehicle (3 times with 10% ethanol in water), and (C) non-labelled H2BPP1 (3 times with 5 μg, dissolved in 10% ethanol in water).

Figure 19 shows (A) survival and (B) average body weight of mice treated with $^{188}$Re-H2BPP1 (3 times with 5 μg, 16 MBq, dissolved in 10% ethanol in water), vehicle (3 times with 10% ethanol in water), or non-labelled H2BPP1 (3 times with 5 μg, dissolved in 10% ethanol in water).

Examples

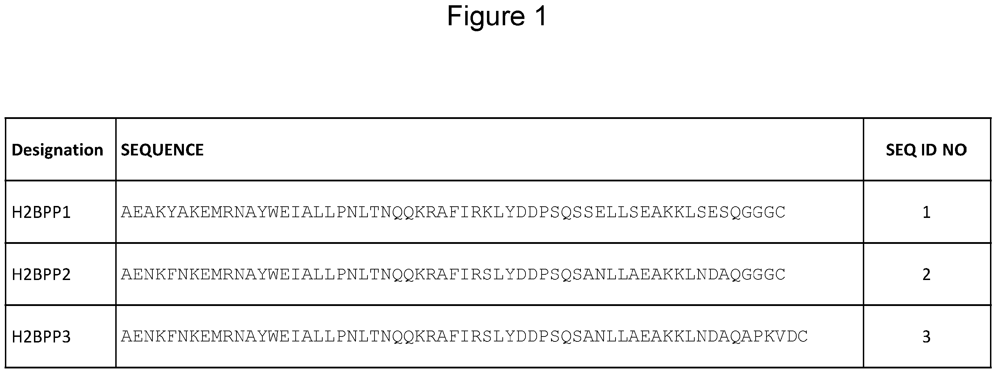

The following Examples disclose the generation and characterization of a novel HER2 targeted polypeptide, H2BPP1 (SEQ ID NO:1), engineered with a cysteine-containing peptide-based chelator (GGGC; SEQ ID NO:5) at the C terminus. The *in vitro* and *in vivo* properties of H2BPP1 were compared with the properties of two previously studied HER2-binding polypeptides: H2BPP2 (SEQ ID NO:2), also containing a C-terminal GGGC chelator, and H2BPP3 (SEQ ID NO:3), containing a C-terminal KVDC chelator (SEQ ID NO:4) (Wållberg et al (2011), J Nucl Med 52:461-469; Ahlgren et al (2010), J Nucl Med 50:781-789). All three polypeptides are identical with regard to the amino acid residues interacting with HER2, but the novel polypeptide H2BPP1 differs in scaffold residues compared to H2BPP2 and H2BPP3. Here, the latter two are identical, apart from the chelating residues at the C-terminus (Figure 1). Because changes in the scaffold might be associated with undesirable changes in properties, for example loss of site-specificity of radiolabeling as well as changes in biodistribution and imaging properties, a clinical translation of a new radiotracer requires a new preclinical evaluation. For the purpose of such evaluation, radiolabeling was here made with Tc-99m. The studies described in the following Examples surprisingly showed that the labeling efficiency and beneficial reduction of kidney uptake was retained despite the change of several amino acids in the scaffold of H2BPP1 in comparison to H2BPP2 and H2BPP3. To conclude, favorable *in vitro* and *in vivo* properties, such as HER2-binding, immunogenicity profile, biodistribution and tumor-targeting, were demonstrated for H2BPP1, making this novel polypeptide suitable for use in diagnostic and therapeutic applications.

Example 1

Production and purity analyses of HER2-binding polypeptides *Methods*

Chemical synthesis of polypeptides: The novel HER2-binding polypeptide H2BPP1 (SEQ ID NO:1) and a reference polypeptide H2BPP2 (SEQ ID NO:2), both comprising a GGGC chelating sequence at the C-terminus, were custom made at Almac (Edinburgh, UK). The polypeptides were produced by means of chemical synthesis, purified by RP-HPLC, exchanged to an acetic acid containing buffer and delivered as a lyophilized product. The lyophilized polypeptides were stored at -20 °C prior to characterization and radiolabeling.

Recombinant production: The reference polypeptide H2BPP3 (SEQ ID NO:3) was produced as described previously (Ahlgren et al (2008), Bioconjugate Chem 19:235-243).

SE-HPLC and RP-UPLC-MS analysis: The purity of H2BPP1 and H2BPP2 was analyzed by Size-Exclusion High-Performance Liquid Chromatography (SE-HPLC) and Reverse Phase Ultra-High-Performance Chromatography Mass Spectrometry (RP-UPLC-MS). The lyophilized polypeptides were dissolved in PBS (2.68 mM KCl, 1.47 mM $KH_2PO_4$, 136.9 mM NaCl, 8.1 mM $Na_2HPO_4$, pH 7.4) supplemented with 2 mM EDTA and incubated for 2 h with occasional swirling. The SE-HPLC analysis was performed on a Superdex peptide GL 10/300 column (Cytiva) connected to an Agilent 1100 HPLC system (Agilent Technologies), at a flow rate of 0.3 ml/min using PBS as running buffer. The RP-UPLC-MS analysis was performed on an Acquity UPLC CSH-C18, 1.7 μm, 2.1 × 150 mm column (Waters) connected to an Agilent 1290 UPLC system equipped with an API-ES and 6130 single quadrupole MSD (Agilent Technologies). Elution was carried out by linear gradient of acetonitrile from 10 to 60 % in 0.1 % TFA during 24 min at flow rate 0.2 ml/min.

*Results*

The peptide synthesis, purification and lyophilization performed well for both H2BPP1 and H2BPP2. The purity of both polypeptides was determined to >99% by SE-HPLC and to >92% by RP-UPLC. The RP-HPLC-MS analysis identified expected masses corresponding to monomeric and Cys-Cys mediated dimeric polypeptides, respectively, at the fractions given in Table 1, based on integration of the 220 nm signal. In conclusion, the respective purities of the two polypeptides were regarded as equivalent and adequate for further *in vitro* and *in vivo* analyses.

*Table 1: Results from RP-UPLC-MS analysis*

| Polypeptide | SEQ ID NO | Monomer (%) | Dimer (%) | Unidentified (%) |
|---|---|---|---|---|
| H2BPP1 | 1 | 93.0 | 2.3 | 4.7 |
| H2BPP2 | 2 | 92.3 | 2.6 | 5.1 |

Example 2

Stability studies of non-labeled HER2-binding polypeptides *Methods*

Circular dichroism spectroscopy: Thermal stability and refolding after heat-induced denaturation were measured for the novel polypeptide H2BPP1 (SEQ ID NO:1) and the reference polypeptide H2BPP2 (SEQ ID NO:2) using circular dichroism spectroscopy. The lyophilized polypeptides were dissolved in PBS and, after 2 h of incubation, sterile filtered through a 0.22 $\mu$m Millex-GV syringe filter (Millipore) and supplemented with EDTA to a final concentration of 2 mM. The polypeptide samples were diluted to 0.5 mg/ml in PBS. Measurements were performed on a Jasco J-810 spectropolarimeter (Jasco Scandinavia AB) using a cell with an optical path-length of 1 mm. For each polypeptide, a first CD spectrum measuring the ellipticity from 250 to 195 nm was recorded at 20 °C. The thermal stability was determined by recording the ellipticity at 221 nm during a variable temperature measurement (VTM; 5 °C/min from 20 °C to 90 °C). After cooling to 20 °C, a second CD spectrum from 250 to 195 nm was recorded.

Long-term stability assessment: The respective stability of H2BPP1 (SEQ ID NO:1) and H2BPP2 (SEQ ID NO:2), lyophilized and stored at -20 °C or dissolved in PBS + 2 mM EDTA and stored at -80 °C and 5 °C, respectively, was monitored for up to 6 months. RP-UPLC and SE-HPLC analyses, performed as described in Example 1, were used for evaluation.

High-temperature stability assessment: H2BPP1 (SEQ ID NO:1) and H2BPP2 (SEQ ID NO:2) were each dissolved in PBS. To each 100 $\mu$g of polypeptide, 56 $\mu$g SnCl2, 75 $\mu$g Na$_4$EDTA and 2.8 mg Na-gluconate were added. The stability of the polypeptides after treatment at the radiolabeling conditions 90 °C for 60 min was evaluated by RP-UPLC and in series with a mass spectrometer (Vion-Q-Tof; Waters). The analysis was performed on an Acquity UPLC CSH-C18, 1.7 $\mu$m, 2.1 × 100 mm column (Waters) at a flow rate of 0.2 ml/min and elution by linear gradient of acetonitrile from 10 to 50 % in 0.1 % formic acid during 24 min.

*Results*

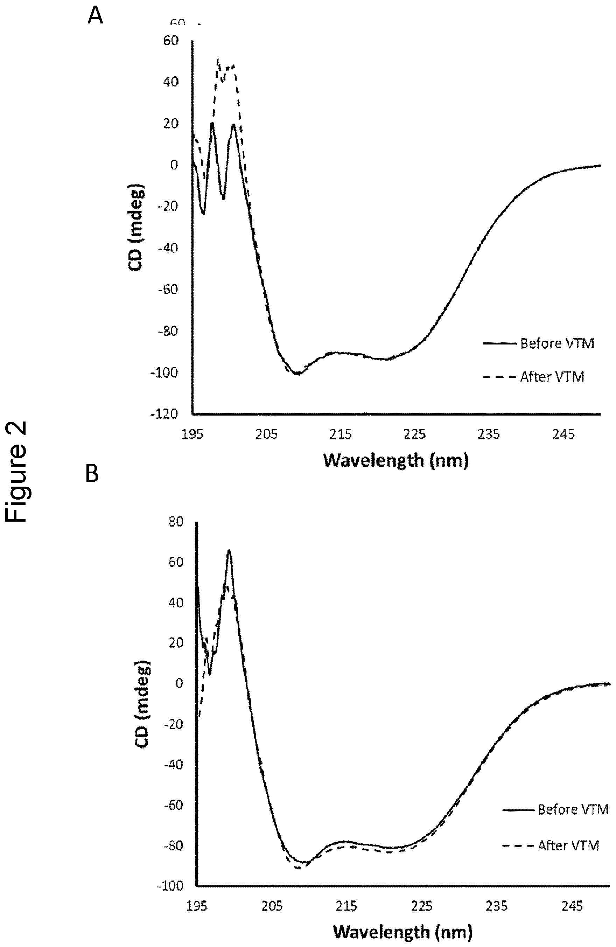

Circular dichroism spectroscopy: The melting temperature Tm, determined from the variable temperature measurements using circular dichroism spectroscopy, was 62 °C for H2BPP1 and 68 °C for H2BPP2. This was considered to be high for Z variant polypeptides. Furthermore, CD spectra measured before and after heat-induced denaturation demonstrated complete refolding and typical alpha helical structures for both polypeptides (Figure 2).

Long-term stability assessment: No difference in stability was detected between H2BPP1 and H2BPP2 when stored lyophilized at -20 °C or dissolved in PBS + 2 mM EDTA and stored at -80 °C or 5 °C for up to 6 months. Table 2 shows a summary of the results from the RP-UPLC.

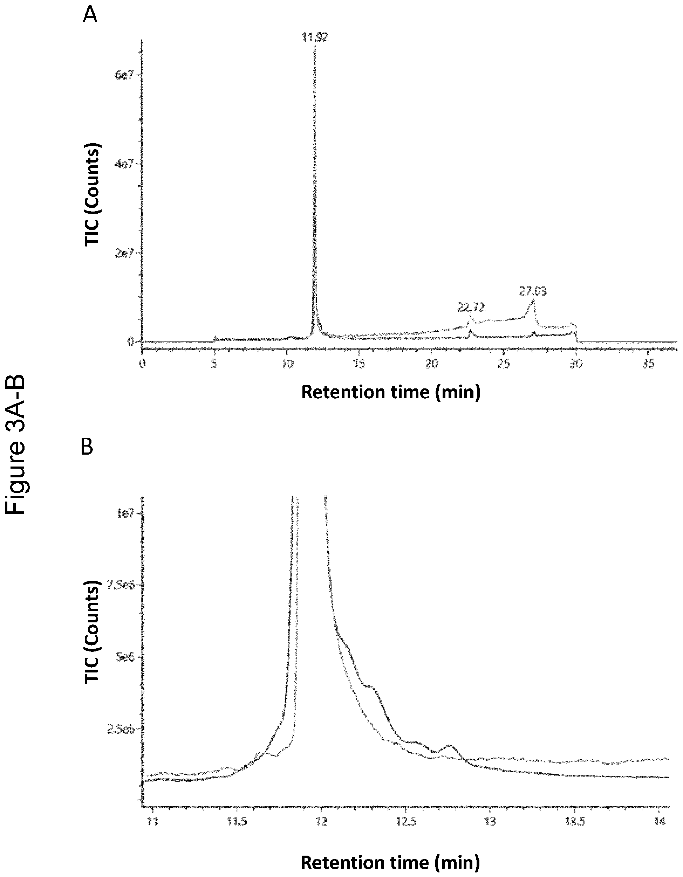

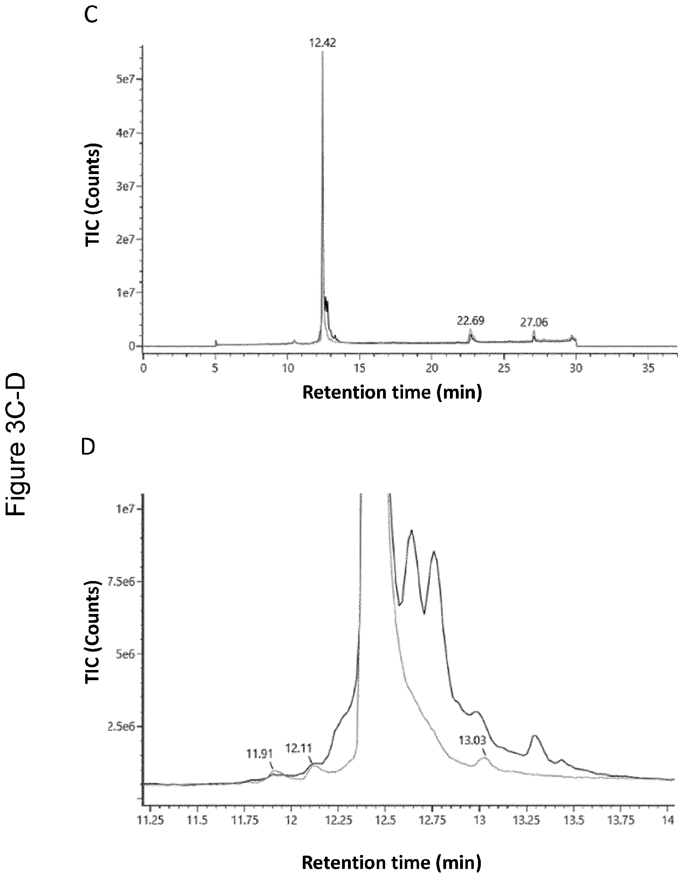

High-temperature stability assessment: RP-UPLC-MS total ion chromatograms (TIC) show that the novel polypeptide H2BPP1 is more stable than the reference polypeptide H2BPP2 after heat treatment at favorable labeling conditions (90 °C for 60 min), as concluded from the fact that more post peak variants where developed for H2BPP2 (compare Figure 3B with Figure 3D). These variants are most likely deamidated polypeptide variants.

*Table 2: RP-UPLC-MS analysis after long-term storage*

| Peptide | Storage conditions | Monomer (%) | Dimer (%) | Unidentified peaks (%) |
|---|---|---|---|---|
| H2BPP1 Lyophilized | -20 °C, 0 mos | 92.9 | 2.3 | 4.8 |
| | -20 °C, 6 mos | 91.8 | 2.6 | 5.6 |

(continued)

| Peptide | Storage conditions | Monomer (%) | Dimer (%) | Unidentified peaks (%) |
|---|---|---|---|---|
| H2BPP1 Dissolved* | -80 °C, 1 mos | 91.1 | 4.1 | 4.8 |
| | -80 °C, 3 mos | 90.2 | 4.2 | 5.6 |
| | -80 °C, 6 mos | 90.9 | 3.9 | 5.2 |
| | +5 °C, 1 mos | 88.4 | 6.5 | 5.1 |
| | +5 °C, 3 mos | 84.8 | 9.3 | 5.9 |
| | +5 °C, 6 mos | 81.8 | 11.5 | 6.7 |
| H2BPP2 Lyophilized | -20 °C, 0 mos | N/A | N/A | N/A |
| | -20 °C, 6 mos | 92.8 | 2.5 | 4.7 |
| H2BPP2 Dissolved* | -80 °C, 1 mos | 90.5 | 4.5 | 5.0 |
| | -80 °C, 3 mos | 92.0 | 5.0 | 3.0 |
| | -80 °C, 6 mos | 91.5 | 4.9 | 3.6 |
| | +5 °C, 1 mos | 88.8 | 6.6 | 4.7 |
| | +5 °C, 3 mos | 88.2 | 8.3 | 3.5 |
| | +5 °C, 6 mos | 85.8 | 9.9 | 4.3 |
| *Dissolved in PBS (pH 7.4) + 2 mM EDTA | | | | |

Example 3

Binding analyses of non-labeled HER2-binding polypeptide

This example presents SPR binding studies performed with the novel polypeptide H2BPP1 (SEQ ID NO:1) to assess its binding affinity and specificity for HER2. Cross-species interactions were studied by analyzing the binding to human, cynomolgus, rat, and mouse HER2, respectively. To confirm the HER2-binding specificity of H2BPP1, binding to the closely related receptor tyrosine kinases HER1 (also known as epidermal growth factor receptor, EGFR), HER3 and HER4 was assessed.

*Methods*

SPR cross-species binding analysis: In a first experiment, the binding affinities of H2BPP1 to Fc chimera proteins with human, cynomolgus, rat and mouse HER2 were measured using a Biacore 8K instrument (Cytiva). Recombinant human ErbB2/HER2-Fc (Sino Biological, cat. no. 10004-H02H), cynomolgus ErbB2/HER2-Fc (Sino Biological, cat. no. 90295-CO2H), rat ErbB2/HER2-Fc (Sino Biological, cat. no. 80079-R02H) and mouse ErbB2/HER2-Fc (Sino Biological, cat. no. 50714-M02H), respectively, was diluted to 10 $\mu$g/ml in immobilization buffer (10 mM sodium acetate, pH 4.5) and immobilized on a CM5 sensor chip (Cytiva) by EDC/NHS coupling chemistry using Amine coupling kit type 2 (Cytiva) according to the manufacturer's instructions. Sensor chip immobilizations resulted in 2342 RU for human, 2066 RU of cynomolgus, 2385 RU of rat and 1674 RU of mouse HER2 Fc chimera proteins, respectively. A single cycle kinetic (SCK) method, allowing a series of analyte injections in one cycle with no regeneration in between, was used for injection of H2BPP1 over the chip surfaces. A blank cycle with HBS-EP+ (10 mM HEPES, 150 mM NaCl, 3 mM EDTA, 0.05 % surfactant P-20, pH 7.4) was run before each analyte cycle for background subtraction. H2BPP1 was successively injected at concentrations of 0.3 and 3 nM during 240 s, followed by a dissociation time of 900 s. The flow rate was 50 $\mu$l/min and HBS-EP+ was used as running buffer. The surfaces were regenerated by one 30 s injection of regeneration buffer (25 mM HCl). The association rate constant ($k_a$), the dissociation rate constant ($k_d$) and the dissociation equilibrium constant ($K_D$) were calculated using the 1:1 binding model of the Biacore 8K Insight Evaluation software.

SPR specificity analysis: In a second experiment, the specificity of H2BPP1 was investigated by analyzing the binding to other members of the ErbB receptor family. Immobilization of recombinant human EGFR/Her1-Fc (R&D Systems, cat. no. 344-ER), ErbB2/HER2-Fc (R&D Systems, cat. no. 1129-ER), ErbB3/Her3-Fc (R&D Systems, cat. no. 348-RB) and ErbB4/Her4-Fc (R&D Systems, cat. no. 1131-ER) and sample analysis was performed as described for the cross-species binding analyses with the exceptions: 1) H2BPP1 was successively injected at concentrations of 0.19, 0.38 and 0.75 nM, respectively, and 2) the dissociation time was 1200 s. Sensor chip immobilizations of human HER Fc chimera proteins resulted in 1592 RU of HER1, 1704 RU of HER2, 2942 RU of HER3 and 2056 RU of HER4.

*Results*

The binding affinity and specificity of the novel H2BPP1 for HER2 was assessed by surface plasmon resonance measurements.

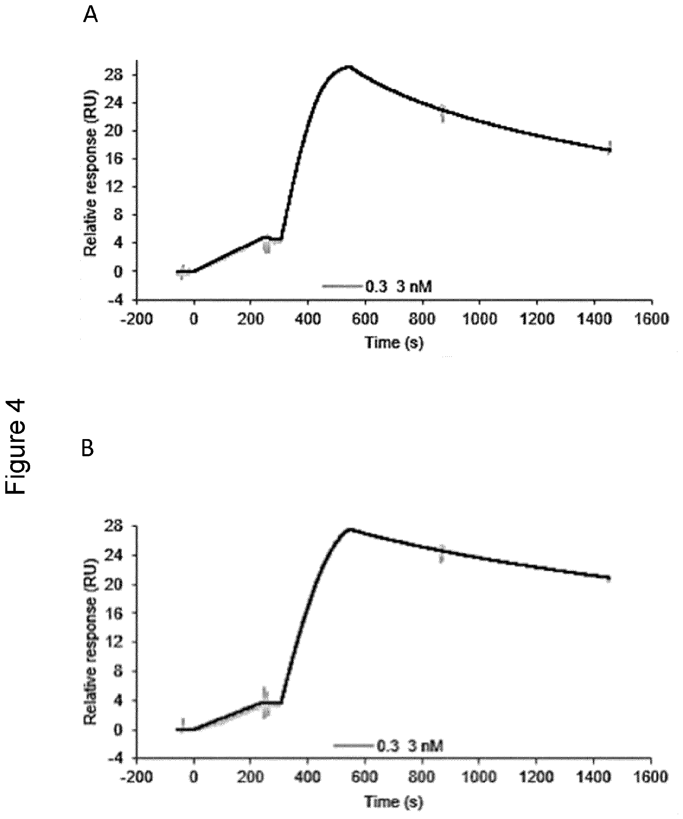

SPR cross-species binding analysis: In a first analysis, H2BPP1 was injected in a SCK concentration series (0.3 and 3 nM) over four Biacore sensor chip channels containing immobilized human, cynomolgus, rat and mouse HER2 Fc chimera protein, respectively. H2BPP1 showed binding to human and cynomolgus HER2 (Figure 4), but not to rat or mouse HER2 (not shown). The association rate constant ($k_a$), the dissociation rate constant ($k_d$) and the dissociation equilibrium constant ($K_D$) for binding of H2BPP1 to human and cynomolgus HER2-Fc, respectively, are summarized in Table 3.

*Table 3: Kinetic data for H2BPP1 binding to human and cynomolgus HER2*

| HER2 species | $k_a$ (M$^{-1}$s$^{-1}$) | $k_d$ (s$^{-1}$) | $K_D$ (M) |
|---|---|---|---|
| Human | $1.1 \times 10^7$ | $1.2 \times 10^{-3}$ | $1.0 \times 10^{-10}$ |
| Cynomolgus | $8.5 \times 10^6$ | $5.1 \times 10^{-4}$ | $6.0 \times 10^{-11}$ |

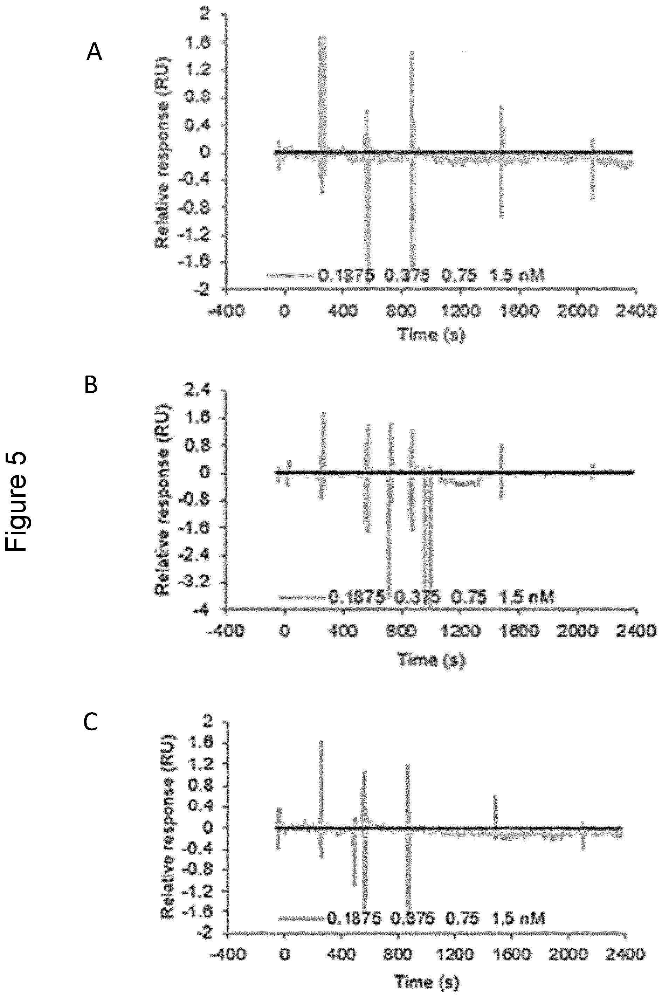

SPR specificity analysis: In a second SPR analysis, the HER2-binding specificity of H2BPP1 was investigated by analyzing the binding to other members of the ErbB receptor family: HER1, HER3 and HER4, respectively. The binding kinetics for human HER2 was in line with the data presented in Table 3, with a $K_D$ determined to 100 pM, whereas no binding was detected to HER1, HER3 or HER4 (Figure 5).

Example 4

*In silico* immunogenicity assessment

This Example describes an *in silico* immunogenicity assessments of the amino acid sequences of the novel HER2-binding polypeptide H2BPP1 and the reference polypeptide H2BPP2, respectively, using a web-based tool for HLA binding predictions. For use in human studies, the least immunogenic polypeptide is highly preferred.

*Methods*

An *in silico* prediction analysis of the novel polypeptide H2BPP1 (SEQ ID NO:1) and the reference polypeptide H2BPP2 (SEQ ID NO:2) was performed using the web-based MHC II binding prediction tool from Immune Epitope Data Base (IEDB, contracted by National Institute of Allergy and Infectious Disease, NIH; http://tools.iedb.org/mhcii/). The tool predicts binding affinities of peptides (by default 15-mers) to selected HLA alleles, and the affinities are ranked in relation to a reference set of peptides. Percentile rank can be used to identify high affinity binding epitopes that are likely to be immunogenic. For background and further details on the IEDB tool, see Vita et al (2019), Nucleic Acids Res 47:339-343. The query parameters used were the IEDB default parameters (method: "IEDB recommended 2.22"; HLA set: full HLA reference set (27 alleles); peptide length: 15-mers). The data was grouped based on percentile rank ($\leq$1 %, 1-2 %, 2-3 %, and 3-10 %) and the number of unique core epitopes (9 amino acids) and alleles per threshold group and query sequence were tallied. Peptides scoring >10 % were classified as insignificant.

*Results*

The potential presence of immunogenic epitopes in the two peptides was evaluated *in silico* using the IEDB web-based MHC II binding prediction tool. The number of predicted distinct core epitopes within the novel polypeptide H2BPP1 and the reference polypeptide H2BPP2, respectively, is presented in Table 4. Any single epitope with affinities to different alleles falling within different thresholds were counted multiple times (once per threshold). Furthermore, the number of distinct HLA alleles were counted and tallied for each sequence and threshold (Table 5). The number of alleles used in the assessment was 27, thus making the maximum score 27.

*Table 4: Number of distinct 9 a.a. core epitopes per threshold group*

| Polypeptide | SEQ ID NO | Threshold ≤1 % | Threshold 1-2 % | Threshold 2-3 % | Threshold 3-10 % |
|---|---|---|---|---|---|
| H2BPP1 | 1 | 1 | 3 | 3 | 12 |
| H2BPP2 | 2 | 1 | 4 | 4 | 15 |

*Table 5: Number of distinct alleles per threshold group*

| Polypeptide | SEQ ID NO | Threshold ≤1 % | Threshold 1-2 % | Threshold 2-3 % | Threshold 3-10 % |
|---|---|---|---|---|---|
| H2BPP1 | 1 | 1 | 3 | 3 | 17 |
| H2BPP2 | 2 | 1 | 4 | 4 | 19 |

Comparing the predicted epitopes within H2BPP1 with those within H2BPP2 reveals that main difference is one 1-2 % threshold epitope predicted to occur at amino acid residue positions 40-48 in the reference polypeptide H2BPP2, but not in the novel polypeptide H2BPP1. In addition, differences with a 3-10 % threshold epitope are one additional epitope at positions 5-13 and one additional epitope at positions 30-52 (where the scaffold residues differ most between the two polypeptides) predicted in the reference polypeptide H2BPP2, but not in the novel polypeptide H2BPP1. To conclude, the novel polypeptide H2BPP1 exhibited a reduced number of potentially immunogenic epitopes compared to the reference polypeptide H2BPP2, which favors H2BPP1 for development for use in humans.

Example 5

Radiolabeling of HER2-binding polypeptides

This Example describes radiolabeling of the novel polypeptide H2BPP1 (SEQ ID NO:1) as well as the two reference polypeptides H2BPP2 (SEQ ID NO:2) and H2BPP3 (SEQ ID NO:3) with the metastable nuclear isomer of technetium-99 ($^{99m}$Tc). Analyses performed to assess the radiochemical yield, purity and stability are also presented.

*Methods*

Reduction of disulfide bonds: Before labeling, each polypeptide was treated with dithiothreitol (DTT; Merck) to reduce any disulfide bond formed between cysteine residues. For this purpose, a solution of DTT (15 µl, 1 M in degassed Milli-Q water) was mixed with the polypeptide (500 µl, 2 mg/ml in degassed PBS). The mixture was incubated at 37 °C for 2 h. The reduced polypeptides were purified using NAP-5 columns (Cytiva) equilibrated in, and eluted with, PBS. Each eluted polypeptide was aliquoted (50 µg in 50 µl) and stored at -80 °C until further use.

Labeling with $^{99m}$Tc: A freeze-dried labeling kit containing 75 µg of tin (II) chloride dihydrate (Fluka Chemika), 5 mg of gluconic acid sodium salt (Celsus Laboratories) and 100 µg of ethylenediaminetetraacetic acid tetra sodium salt (EDTANa$_4$) (Sigma-Aldrich) was prepared as described previously (Ahlgren et al (2010), Nucl Med Biol 37:539-546). $^{99m}$Tc was obtained as pertechnetate by elution of Ultra TechneKow generator (Mallinckrodt) with sterile 0.9 % sodium chloride (Mallinckrodt). Radiolabeling of each polypeptide was performed by adding the contents of the freeze-dried kit, dissolved in 100 µl degassed PBS, to 50 µg of polypeptide. To the reaction mixture, 100 µl (150-250 MBq) of the generator eluted $^{99m}$Tc-pertechnetate was added and the vial was degassed to protect the mixture from oxidation. The reaction vial was thoroughly vortexed and incubated at 90 °C for 1 h.

Evaluation of radiolabeling: The radiochemical yield of each polypeptide was analyzed by instant thin-layer chromatography using ITLC-SG strips (Agilent Technologies) developed with PBS (polypeptide: retention factor (Rf)=0.0; other forms of $^{99m}$Tc: Rf=1.0). The reduced hydrolyzed technetium colloid (RHT) level in the product was measured using pyridine:acetic acid:water (5:3:1.5) as the mobile phase ($^{99m}$Tc colloid: Rf=0.0, other forms of $^{99m}$Tc and radiolabeled polypeptide: Rf=1.0). Since the radiochemical yield was >95 % for all conjugates, no further purification was performed. The results of the ITLC measurement were validated by SDS-PAGE using NuPAGE 4-12 % Bis-Tris Gel in MES buffer (both from Invitrogen). The Cyclone Storage Phosphor system (Perkin-Elmer) was used for quantitative measurement of radioactivity distribution in both the ITLC strips and electrophoresis gels.

To cross-validate radio-ITLC data further, RP-HPLC was conducted on a LaChrom Elite® system (VWR-Hitachi) equipped with an L-2130 pump, a UV detector (L-2400) and a radiation flow detector (Bioscan) coupled in series. Purity analysis of $^{99m}$Tc-labeled compounds was performed using an analytical column (Luna® 5 µm C18, 100 Å; 150 × 4.6 mm column; Phenomenex). The RP-HPLC conditions were as follows: Solvent A: 10 mM TFA in Milli-Q-water; Solvent B: 10 mM TFA in acetonitrile; UV-detection at 220 nm; gradient elution: 0-15 min at 5 to 70 % B, 15-18 min at 70 to 95 % B, 19-20 min at 5 % B; and the flow rate was 1.0 ml/min.

To evaluate the *in vitro* stability of the radiolabeled conjugate, fractions of each of the freshly labeled polypeptides (10 μl, 0.4 μg) were incubated with an excess amount of PBS (40 μl) for 1 and 4 h at 37 °C. The test was run in triplicates. The release of $^{99m}$Tc was monitored by ITLC as described above.

*Results*

All three polypeptide variants were successfully labeled with **$^{99m}$Tc,** with both the radiolabeling yield and radiochemical purity exceeding 95 %. All conjugates were stable during incubation at 37 °C for 4 h in the presence of excess PBS. Less than 3 % release of $^{99m}$Tc was observed. The results of radiolabeling and *in vitro* stability tests are summarized in Table 6.

Table 6: Results of $^{99m}$Tc *radiolabeling and* in *vitro stability*

| Polypeptide | SEQ ID NO | Radiolabeling yield (%) | Radiochemical purity (%) | Stability in PBS (%) | |
|---|---|---|---|---|---|
| | | | | **1 h** | **4 h** |
| H2BPP1 | 1 | 95.6 ± 2.7 | > 95 | 98.7 ± 0.7 | 97.5 ± 0.9 |
| H2BPP2 | 2 | 96.3 ± 1.9 | > 95 | 98.1 ± 0.2 | 97.2 ± 0.3 |
| H2BPP3 | 3 | 98.6 ± 1.4 | > 95 | 96.2 ± 0.8 | 97.1 ± 0.4 |

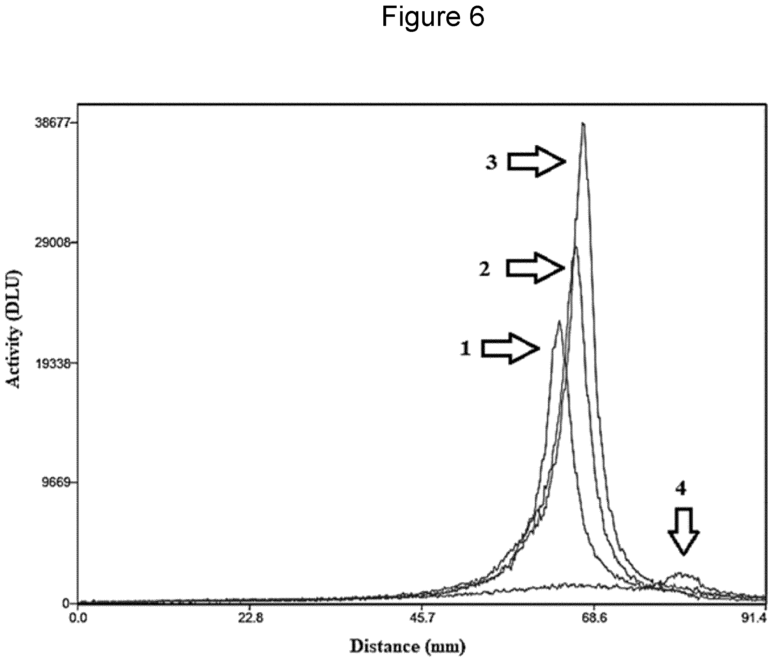

SDS-PAGE, RP-HPLC and analysis was performed to cross-validate the ITLC data. The SDS-PAGE analysis (Figure 6) showed no indications of aggregation, decomposition or release of radionuclide, since no radioactivity bands of higher or lower molecular weight could be observed. In the radio RP-HPLC analysis, the retention time of all polypeptides was around 8.6-9.2 min. The retention time of labeled polypeptide (measured by radioactivity detector) was the same as for non-labeled polypeptide (measured by UV detector). This confirms the authenticity of the labeled peptides.

Example 6

*In vitro* characterization of radiolabeled HER2-binding polypeptides

This Example describes characterization, using *in vitro* cell based assays, of the three $^{99m}$Tc-labeled polypeptides prepared as described in Example 5.

*Materials*

Cell cultures: HER2-expressing ovarian carcinoma SKOV-3 and breast carcinoma BT-474 cell lines obtained from the American Type Culture Collection (ATCC) were used. Cells were cultured in RPMI medium (Flow Irvine) supplemented with 10 % fetal calf serum, 2 mM L-glutamine, and PEST composed of 100 IU/ml penicillin and 100 mg/ml streptomycin (referred to as "complete medium" in the following). Cells were seeded in the cell-culture dishes at a density of $10^6$ cells/dish. A set of three dishes was used for each data point in the *in vitro* studies of binding specificity, cellular retention and cellular processing.

*In vitro* binding specificity: In control dishes, SKOV-3 and BT-474 cells were pre-saturated with a 500-fold excess of non-labeled H2BPP3 for 15 min. 0.5 nM of the respective labeled polypeptide $^{99m}$Tc-H2BPP1 (novel), $^{99m}$Tc-H2BPP2 (reference) and $^{99m}$Tc-H2BPP3 (reference) were added to test and control dishes, respectively, and the cells were incubated in a humidified incubator (5 % $CO_2$, 37 °C) for 1 h. The medium was discarded and the cells were washed with cold serum-free medium, before trypsin-EDTA solution (0.5 ml per dish) was added and cells incubated for an additional 10 min. Detached cells were diluted with 0.5 ml complete medium, re-suspended and transferred to fraction tubes. The radioactivity of cells was measured using an automated gamma counter with an NaI(TI) detector (1480 Wizard, Wallac) and the cell-bound radioactivity was calculated.

*In vitro* binding affinity: The binding kinetics of the $^{99m}$Tc-labeled polypeptides H2BPP1 (novel), H2BPP2 (reference) and H2BPP3 (reference) to HER2 receptors were measured using a LigandTracer Yellow instrument (Ridgeview Instruments). SKOV-3 cells were seeded on a local area of a cell culture dish (Nunclon™, Size 100620, NUNC A/S). The measurements were performed at room temperature (RT) to prevent internalization. Uptake curves were recorded at 0.33, 1 and 3 nM of $^{99m}$Tc-labeled polypeptides, after which the radioactive medium was withdrawn, fresh non-radioactive medium was added and the dissociation curve was recorded. The data was analyzed using the Interaction Map software (Ridgeview Diagnostics) to calculate association rate, dissociation rate and dissociation constant at equilibrium ($K_D$). Analysis was performed in duplicates.

Cellular processing studies: Cellular processing was studied using previously described and validated methods (Wållberg and Orlova (2008), Cancer Biother Radiopharm 23:435-442). In brief, SKOV-3 and BT-474 cells ($1 \times 10^6$ cells/dish) were incubated with 0.5 nM of labeled H2BPP1 (novel), H2BPP2 (reference) and H2BPP3 (reference), respectively, at RT for 1 h. Thereafter, the medium was removed, the cells were washed, new medium was added and the cells were placed in a humidified incubator at 37 °C. At 0, 1, 2, 4 and 6 h after incubation, the internalized fraction was determined by an acid wash method. The membrane-bound polypeptides were removed from cells by treatment with 4 M urea solution in a 0.1 M glycine buffer, pH 2.5, for 5 min on ice. The cell debris containing the internalized conjugates was detached by treatment with 1 M NaOH. The radioactivity of cells was measured using an automated gamma counter with an NaI(TI) detector (1480 Wizard, Wallac), and the percentage of membrane-bound and internalized radioactivity was calculated.

*Results*

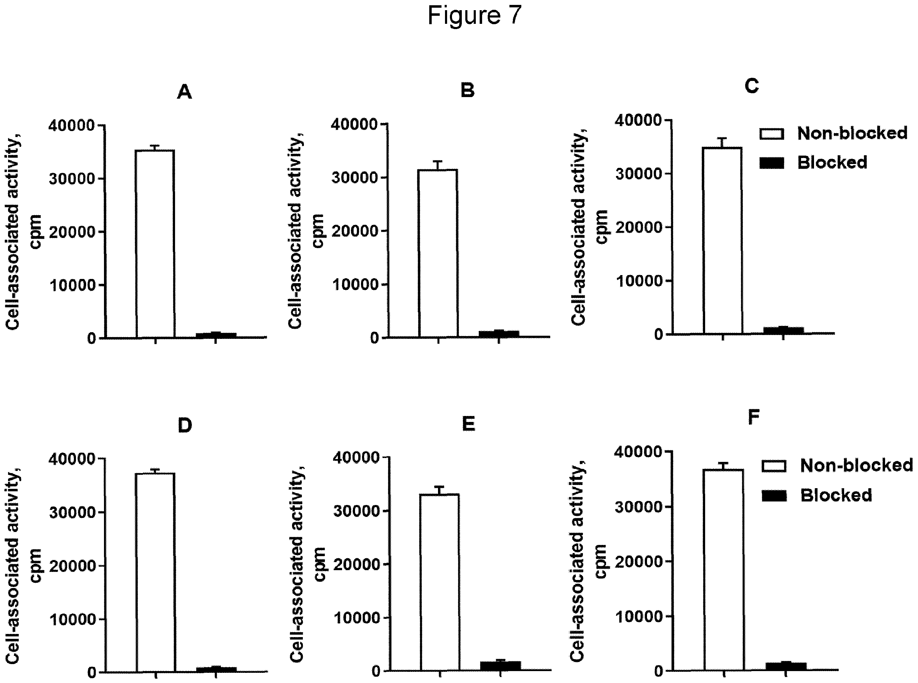

*In vitro* binding specificity: HER2-binding specificity of the H2BPP1 (novel), H2BPP2 (reference) and H2BPP3 (reference) polypeptides was evaluated using a saturation experiment. The binding was significantly ($p < 5 \times 10^{-6}$) decreased when the cells were pre-saturated with the non-labeled H2BPP3 (Figure 7), confirming that the binding of both novel and reference polypeptides was HER2-mediated.

*In vitro* binding affinity: The binding kinetics of the $^{99m}$Tc-labeled polypeptides H2BPP1 (novel), H2BPP2 (reference) and H2BPP3 (reference), respectively, to HER2 receptors on SKOV-3 cells measured using a LigandTracer are summarized in Table 7. The best fit of the binding of the all conjugates to SKOV-3 cell line was achieved using a 1:1 model. Interaction Map calculation showed a rapid association followed by very slow dissociation for all conjugates, resulted in picomolar dissociation constants ($K_D$) at equilibrium.

*Table 7: Apparent kinetic parameters of $^{99m}$Tc-labeled polypeptides interacting with HER2-expressing SKOV-3 cells*

| Polypeptide | SEQ ID NO | $k_a$ (M$^{-1}$s$^{-1}$) | $k_d$ (s$^{-1}$) | $K_D$ (M) |
|---|---|---|---|---|
| $^{99m}$Tc-H2BPP1 | 1 | $[3.7\pm0.1] \times 10^4$ | $[2.2\pm0.05] \times 10^{-6}$ | $[58\pm2] \times 10^{-12}$ |
| $^{99m}$Tc-H2BPP2 | 2 | $[2.4\pm0.8] \times 10^4$ | $[2.0\pm0.10] \times 10^{-6}$ | $[90\pm22] \times 10^{-12}$ |
| $^{99m}$Tc-H2BPP3 | 3 | $[2.9\pm0.3] \times 10^4$ | $[1.5\pm0.05] \times 10^{-6}$ | $[53\pm4] \times 10^{-12}$ |

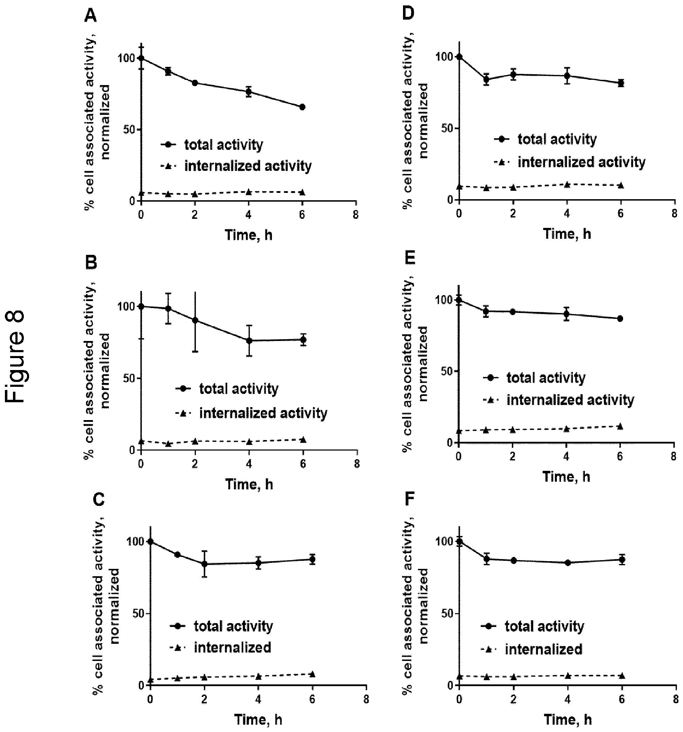

Cellular processing studies: The results of the cellular retention and internalization experiment showed slow internalization of all polypeptides (Figure 8). The reference polypeptide $^{99m}$Tc-H2BPP3 showed a slight tendency towards a higher retention after 6 h incubation (87.6±3.5 and 87.3±3.5 % for SKOV-3 and BT-474 cells, respectively) than the polypeptides containing the GGGC chelator, i.e. the novel polypeptide $^{99m}$Tc-H2BPP1 (65.8±2.1 and 81.5±2.3% for SKOV-3 and BT-474, respectively) and the reference polypeptide $^{99m}$Tc-H2BPP2 (76.8±4.0 and 86.9±1.2 %, for SKOV-3 and BT-474, respectively).

Conclusion: The novel polypeptide $^{99m}$Tc-H2BPP1 was shown to bind specifically to HER2 expressing cells with an affinity ($K_D$) of 58±2 pM, which is favorable for retention of activity in tumors despite a slow internalization rate.

Example 7

*In vivo* biodistribution studies

*Methods*

Animal handling: Animal experiments were performed in accordance with the national legislation for work with laboratory animals. Approval was granted by the Ethical Committee for Animal Research in Uppsala. Groups of four mice per data point were used in these experiments.

Biodistribution in non-tumor-bearing mice: To ensure that the scaffold re-engineering of the novel polypeptide $^{99m}$Tc-H2BPP1 did not have strong adverse effect on its *in vivo* behavior, biodistribution in female NMRI mice at 4 h after injection was compared with biodistribution of the reference polypeptides $^{99m}$Tc-H2BPP2 and $^{99m}$Tc-H2BPP3. Non-tumor-bearing mice (weight: 25.5±2.4 g) were injected with 1 μg of respective $^{99m}$Tc-labeled polypeptide (60 kBq, 100 μl in PBS) into the tail vein. After 4 h, mice were euthanized by overdosing of anesthesia (Rompun/Ketalar) followed by heart puncture, and blood samples were collected. Organs and tissue samples were collected and weighed. The organ radioactivity was measured using a gamma-spectrometer with a NaI(TI) detector (1480 Wizard, Wallac), and uptake values for organs were calculated as percentage injected dose per gram tissue (% ID/g).

Biodistribution in tumor-bearing mice: Biodistribution and targeting properties were evaluated in BALB/C nu/nu mice bearing HER2-positive SKOV-3 xenografts. To establish xenografts, $10^7$ SKOV-3 cells were subcutaneously injected into the right hind leg of female BALB/c nu/nu mice. As a specificity control, HER2-negative Ramos xenografts were used. $5 \times 10^6$ Ramos cells (ATCC) were subcutaneously implanted on the left hind leg of female BALB/c nu/nu in 5 mice. The experiments were performed two weeks after cell implantation. The average animal weight was $18.2 \pm 0.7$ g. The average tumor weight was $0.06 \pm 0.02$ and $0.03 \pm 0.01$ g for SKOV-3 and Ramos xenografts, respectively. The biodistribution of $^{99m}$Tc-H2BPP1 was measured 1, 4, 8 and 24 h after injection in mice bearing SKOV-3 xenografts. Three groups of tumor-bearing mice were injected with 4 $\mu$g of $^{99m}$Tc-H2BPP1 (80 kBq, 100 $\mu$l in PBS) into the tail vein. For measurement of biodistribution at 24 h after injection, one group of mice was injected with 640 kBq, but the injected protein mass was the same, 4 $\mu$g. To evaluate the HER2-specificity of $^{99m}$Tc-H2BPP1 accumulation in tumors, one group of animals bearing HER2-negative Ramos xenografts (average mouse weight was $17.6 \pm 1.2$ g) was injected with 4 $\mu$g of $^{99m}$Tc-H2BPP1 (80 kBq, 100 $\mu$l in PBS), and the biodistribution was measured 4 h after injection. For comparison, one group of mice was injected with the reference polypeptide $^{99m}$Tc-H2BPP2 (the same activity and dosing as for $^{99m}$Tc-H2BPP1), and biodistribution was measured 4 h after injection. The measurement of biodistribution in tumor-bearing mice was performed as described above for NMRI mice.

*In vivo* imaging: To confirm the biodistribution results, a small animal SPECT/CT imaging was performed. One SKOV-3 xenograft mouse and one Ramos xenograft mouse were intravenously injected with 20 MBq/4 $\mu$g of $^{99m}$Tc-H2BPP1. The mice were imaged at 4 h after injection using a nanoScan SPECT/CT scanner (Mediso Medical Imaging Systems). The mice were euthanized by $CO_2$ asphyxiation immediately before being placed in the camera. The computed tomography (CT) acquisition was carried out at the following parameters: energy peak of 50 kV, 670 $\mu$A, 480 projections, 2.29 min acquisition time. SPECT acquisition was performed at the following parameters: $^{99m}$Tc energy peak of 140 keV, window width of 20 %, matrix of 256x256, acquisition time of 1 h. CT images were reconstructed in real-time using Nucline 2.03 Software (Mediso Medical Imaging Systems). Raw SPECT data were reconstructed using Tera-Tomo™ 3D SPECT reconstruction technology.

*Results*

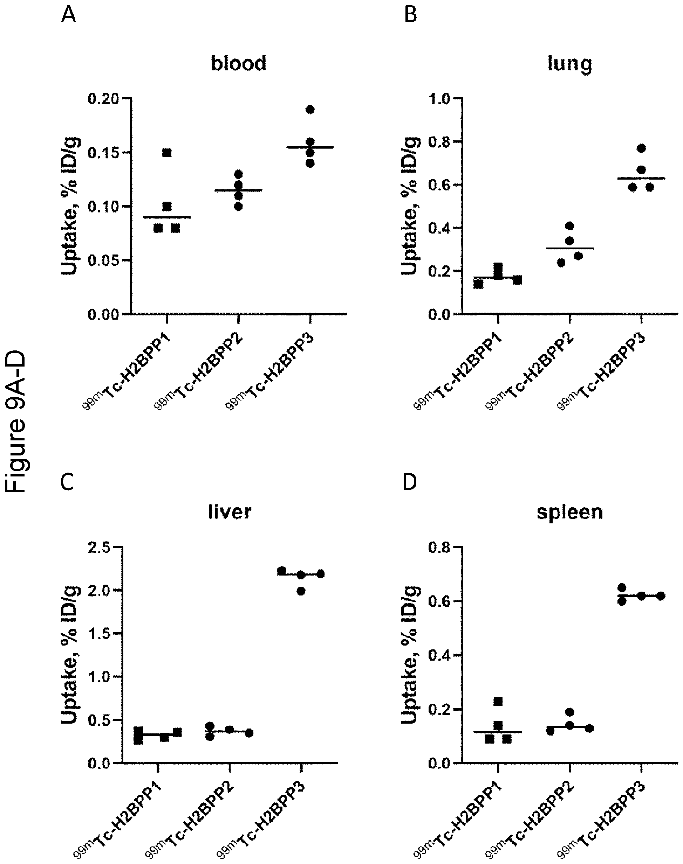

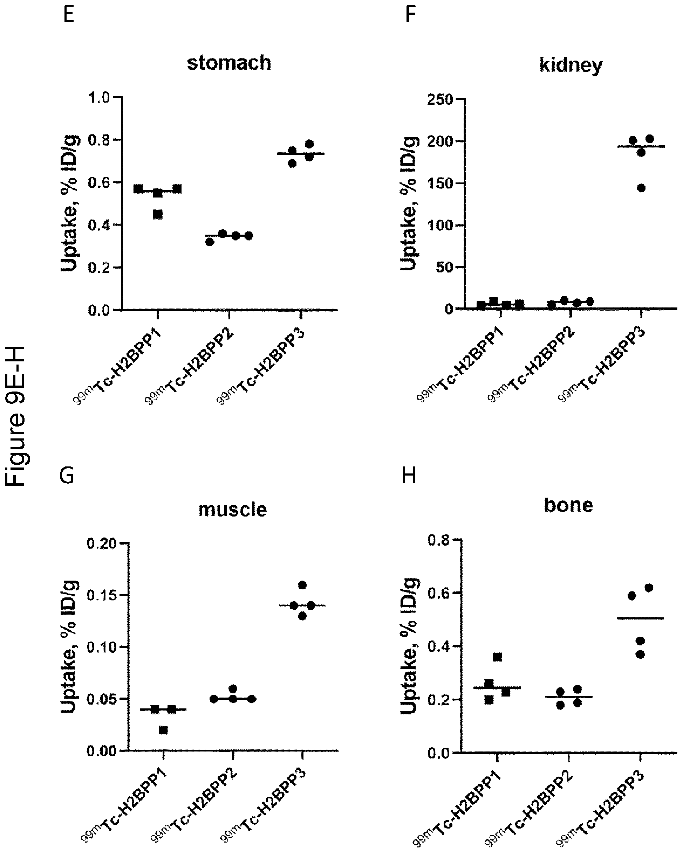

Biodistribution in non-tumor-bearing mice: Comparison of the biodistribution in NMRI mice 4 h after injection of the novel polypeptide $^{99m}$Tc-H2BPP1 and the two reference polypeptides $^{99m}$Tc-H2BPP2 and $^{99m}$Tc-H2BPP3 is presented in Figure 9. The biodistribution data of $^{99m}$Tc-H2BPP2 and $^{99m}$Tc-H2BPP3 were in excellent agreement with the data obtained previously for these reference polypeptides (Wållberg *et al* (2011), *supra;* Ahlgren *et al* (2010), *supra)*. The renal uptake of the GGGC-containing polypeptides $^{99m}$Tc-H2BPP1 ($5.9 \pm 2.1$ %ID/g) and $^{99m}$Tc-H2BPP2 ($7.9 \pm 2.1$ %ID/g) was significantly lower (p < 0.0005) than for $^{99m}$Tc-H2BPP3 ($183.8 \pm 27.3$ %ID/g). In other words, the renal uptake for $^{99m}$Tc-H2BPP1 and $^{99m}$Tc-H2BPP2 was 25-30 fold lower compared to that of $^{99m}$Tc-H2BPP3. Furthermore, $^{99m}$Tc-H2BPP1 and $^{99m}$Tc-H2BPP2 provided significantly (p < 0.05) lower uptakes in lung, liver, spleen, stomach, muscle and bone than $^{99m}$Tc-H2BPP3. The uptake of $^{99m}$Tc-H2BPP1 and $^{99m}$Tc-H2BPP2 in liver was almost four-fold lower compared to the uptake of $^{99m}$Tc-H2BPP3. Such lower uptake is a favorable feature, because it results in a reduced background activity for imaging of liver metastases, which are frequent in breast cancer. The novel polypeptide $^{99m}$Tc-H2BPP1 also showed significantly lower blood concentration than $^{99m}$Tc-H2BPP3.

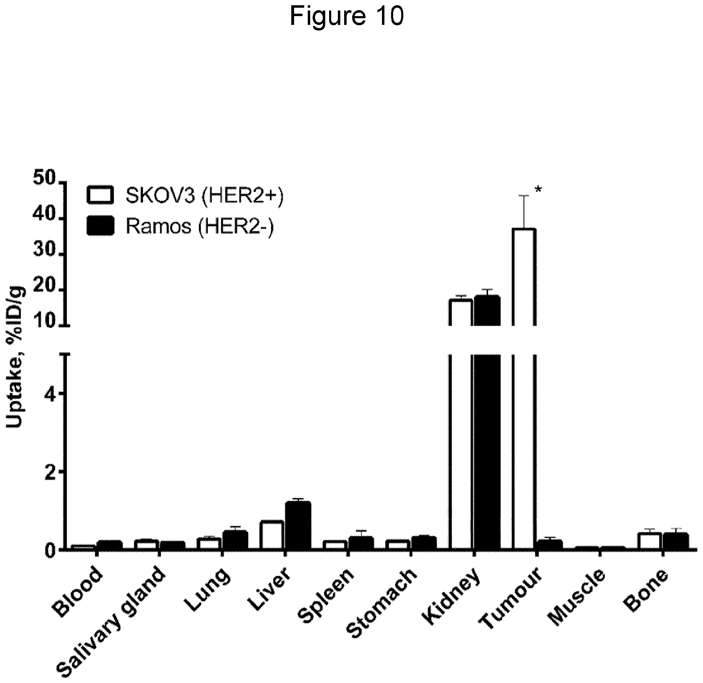

Biodistribution in tumor-bearing mice: The experiments performed in nude mice bearing human cancer xenografts demonstrated that the uptake of $^{99m}$Tc-H2BPP1 in HER2-expressing SKOV-3 xenografts was 175-fold higher (p < 0.0005) than in HER2-negative Ramos xenografts 4 h after injection (Figure 10), which confirmed that the tumor accumulation was HER2-specific. The biodistribution data of the novel polypeptide $^{99m}$Tc-H2BPP1 at 1, 4, 8 and 24 h after injection is presented in Table 8. $^{99m}$Tc-H2BPP1 showed efficient targeting, as the tumor uptake was $24 \pm 7$ %ID/g already 1 h after injection. Despite the fact that it has been shown that the $[^{99m}$Tc](Tc=O)-GGGC complex acts as a non-residualizing label, the retention of activity in tumor over time was good. Without wishing to be bound by theory, the very high affinity of the binding polypeptide for HER2 provides the basis for these beneficial properties. There was no significant difference between tumor uptake at 1 and 8 h after injection, and the tumor uptake decreased only twofold by 24 h after injection. On the contrary, clearance from normal tissues was rapid. The activity associated with kidneys declined very quickly over time ($40 \pm 1$ %ID/g for 1 h p.i., compared to $5.3 \pm 0.3$ % ID/g for 24 h p.i.). Such a biodistribution pattern results in favorably high tumor-to-organ ratios of $^{99m}$Tc-H2BPP1 already at 4 h after injection (Table 9).

*Table 8. Biodistribution of $^{99m}$Tc-H2BPP1 in BALB/C nu/nu mice bearing SKOV-3 xenografts. Data expressed as %ID/g; averages from 4 mice ± SD*

| Organ | 1 h | 4 h | 8h | 24 h |
|---|---|---|---|---|
| Blood | 1.6±0.2 | 0.102±0.004 | 0.08±0.03 | 0.06±0.02 |
| Salivary gland | 0.8±0.1 | 0.23±0.05 | 0.07±0.01 | 0.06±0.02 |
| Lung | 2.7±0.6 | 0.28 ±0.07 | 0.14±0.01 | 0.11±0.02 |
| Liver | 2.0±0.4 | 0.71±0.04 | 0.48±0.08 | 0.42±0.05 |
| Spleen | 0.9±0.1 | 0.21±0.02 | 0.17±0.06 | 0.18±0.02 |
| Stomach | 1.4±0.3 | 0.22±0.03 | 0.13±0.03 | 0.11±0.03 |
| Kidney | 40±1 | 17±1 | 9±1 | 5.3±0.3 |
| Tumor | 24±7 | 37±9 | 23±6 | 13±2 |
| Muscle | 0.5±0.1 | 0.06±0.02 | 0.04±0.02 | 0.03±0.02 |
| Bone | 1.4 ±0.4 | 0.4±0.1 | 0.21±0.04 | 0.27±0.02 |
| Uterus | 1.8 ±0.5 | 0.20±0.08 | 0.08±0.04 | 0.10±0.03 |
| Brain | 0.06±0.01 | 0.0110±0.0005 | 0.009±0.002 | 0.009±0.003 |
| Pancreas | 0.48±0.01 | 0.054±0.001 | 0.05±0.01 | 0.05±0.01 |
| Small intestine | 1.2±0.3 | 0.13±0.07 | 0.12±0.03 | 0.08±0.02 |

*Table 9. Tumor-to-organ ratios for $^{99m}$Tc-H2BPP1 in BALB/C nu/nu mice bearing SKOV-3 xenografts. Data expressed as %/ID/g; averages from 4 mice ± SD*

| Organ | 1h | 4h | 8h | 24h |
|---|---|---|---|---|
| Blood | 16±6 | 363±84 | 284±85 | 250±9 |
| Salivary gland | 29±6 | 161±27 | 325±118 | 220±53 |
| Lung | 9±2 | 136±49 | 170±51 | 127±20 |
| Liver | 12±1 | 52±11 | 50±20 | 32±3 |
| Spleen | 27±7 | 172±34 | 148±66 | 75±10 |
| Stomach | 18±5 | 165±23 | 177±38 | 138±14 |
| Kidney | 0.6±0.2 | 2.1±0.5 | 2.8±0.9 | 2.4±0.4 |
| Muscle | 52±8 | 709±300 | 527±95 | 538±324 |
| Bone | 18±1 | 89±12 | 115±46 | 48±9 |
| Uterus | 14±4 | 232±178 | 315±233 | 137±37 |
| Brain | 423±155 | 3397±947 | 2629±947 | 1548±684 |
| Pancreas | 51±15 | 690±175 | 477±193 | 276±40 |
| Small intestine | 21±4 | 288±103 | 209±127 | 166±49 |

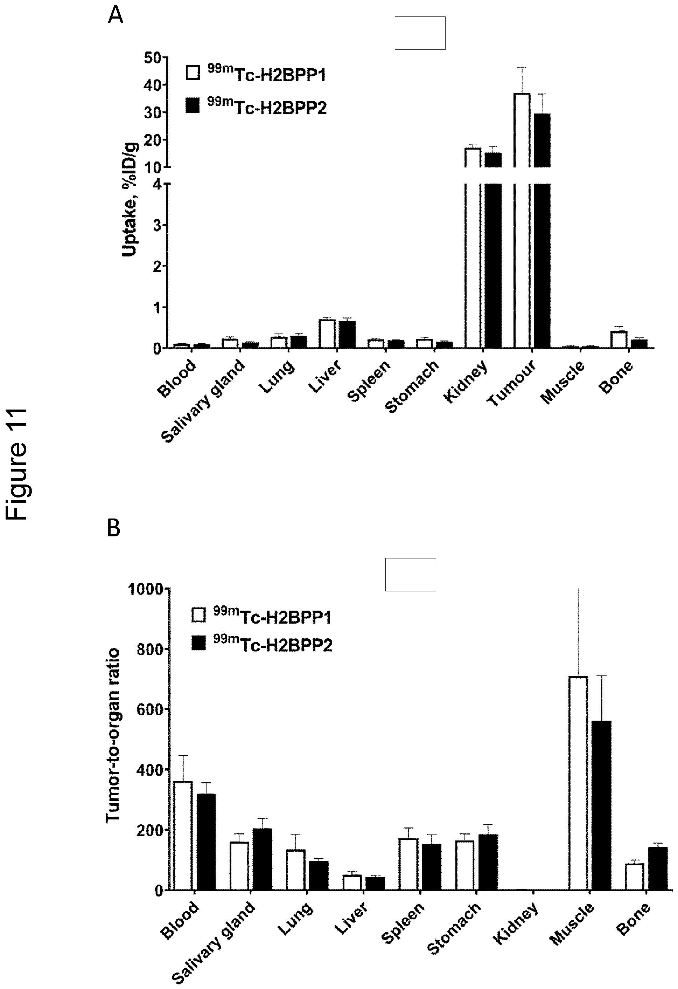

Direct comparison of biodistribution of $^{99m}$Tc-H2BPP1 and $^{99m}$Tc-H2BPP2 in the same batch of nude mice bearing human SKOV-3 xenografts 4 h after injection (Figure 11) demonstrated that the biodistribution of these polypeptides is very similar, i.e. the scaffold changes in the novel polypeptide do not have any negative impact on the biodistribution. Furthermore, there was no significant difference in tumor-to-organ ratios between the two conjugates, except for the tumor-to-bone ratio. The tumor-to-kidney ratio for both conjugates was approximately on the same level (2.2 ±0.5 and 1.9±0.2, for $^{99m}$Tc-H2BPP1 and $^{99m}$Tc-H2BPP2, respectively).

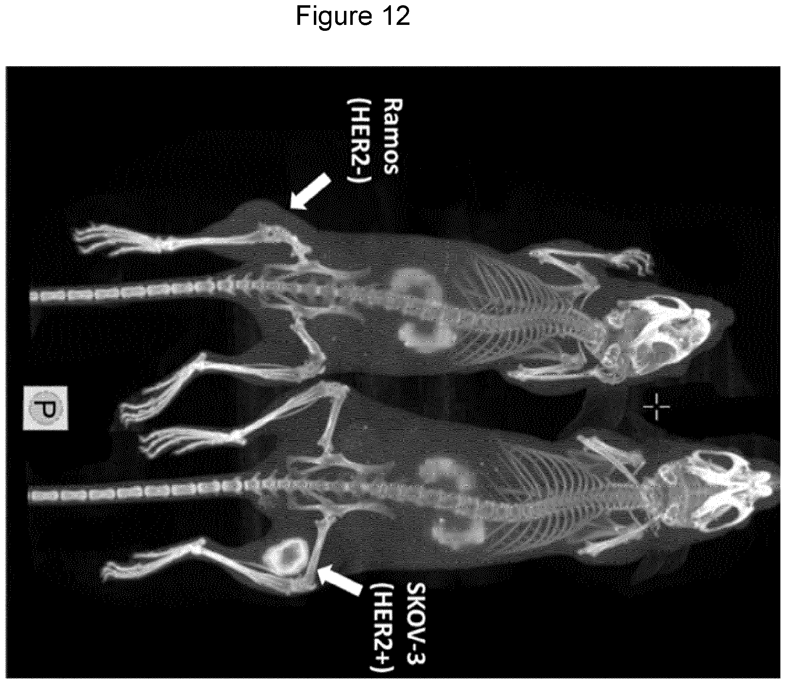

*In vivo* imaging: Results of the microSPECT/CT imaging (Figure 12) demonstrated that a high-contrast visualization of HER2 expression in HER2 positive tumor using $^{99m}$Tc-H2BPP1 is feasible. The SKOV-3 tumor on the right hind leg was visualized clearly and with a high contrast at 4 h after injection. In agreement with the biodistribution data, the radioactivity uptake in the tumor was considerably higher than in the kidney. As expected, the radioactivity uptake in HER2-negative Ramos xenografts was much lower than in the HER2-positive SKOV-3 xenograft.

*Conclusion*

The extensive re-engineering of the scaffold of the novel polypeptide H2BPP1 did not compromise the imaging properties of this polypeptide labelled with [99mTc] using the GGGC chelator engineered within its C-terminus. This new probe, [99mTc]-H2BPP1, provided an excellent tumor-to-blood ratio, also compared to the HER2-imaging probes for single photon emission computed tomography (SPECT) described in the literature so far. We conclude that [99mTc]-H2BPP1 is a promising candidate for further clinical translation studies, as it provides high tumor-to-organ ratios and a low renal uptake.

Example 8

Dosimetry evaluation for human use

*Methods*

To evaluate dosimetry in humans for the novel polypeptide [99mTc]-H2BPP1, uptake values in mice were scaled up using the well-established "percent kg/g method" (Stabin (2008), Fundamentals of Nuclear Medicine Dosimetry, New York, NY: Springer; p 83-86), according to the following:

$$(\%IA/organ)_{human} = [(\%IA/g)_{animal} \times (kg_{TBweight})_{animal} \times (g_{organ}/(kg_{TBweight})_{human}]$$

wherein "%IA" represents percent injected activity, and "TBweight" represents total body weight.

Organ uptakes for human were calculated using organ weights of the reference adult female (Report of the Task Group on Reference Man, ICRP Publication 23 (1975). Pergamon Press, Oxford). Residence times were calculated as the area under the curve of exponential fits for human time-activity curves. Remainder of the body residence time was based on radioactivity in carcass. Absorbed doses were estimated using the OLINDA/EXM 1.0 software.

*Results*

Estimated absorbed doses of the novel polypeptide [99mTc]-H2BPP1 in humans are given in Table 10. According to calculations using OLINDA/EXM 1.0, the effective dose should be 0.00066 mSv/MBq (Effective Dose equivalent: 0.00116 mSv/MBq).

*Table 10. Calculated absorbed dose (mGy/MBq) for [99mTc]-H2BPP1 in humans using OLINDA/EXM 1.0*

| Organ | Absorbed dose (mGy/MBq) | Organ | Absorbed dose (mGy/MBq) |
|---|---|---|---|
| Adrenals | 0.0009 | Muscle | 0.00034 |
| Brain | 0.00016 | Ovaries | 0.00042 |
| Breasts | 0.00029 | Pancreas | 0.00073 |
| Gallbladder wall | 0.0007 | Red marrow | 0.00062 |
| Lower large intestine wall | 0.0004 | Osteogenic cells | 0.0026 |
| Small intestine | 0.00049 | Skin | 0.00021 |
| Stomach wall | 0.00052 | Spleen | 0.00078 |
| Upper large intestine wall | 0.0005 | Thymus | 0.00049 |
| Heart wall | 0.00160 | Thyroid | 0.00029 |
| Kidney | 0.0069 | Urinary bladder | 0.00037 |
| Liver | 0.00088 | Uterus | 0.00051 |
| Lungs | 0.0007 | Total body | 0.00048 |

Example 9

Radiolabeling of H2BPP1 with $^{188}$Re

*Methods*

Labeling of polypeptide with $^{188}$Re: $^{188}$Re was obtained as perrhenate by elution of a $^{188}$W/$^{188}$Re generator with 4 ml sterile 0.9 % sodium chloride (both from OncoBeta GmbH, Germany). Radiolabeling was performed in two alternative ways: 1) with a low eluate volume (1500 μl or less) or 2) with a high eluate volume (4000 μl).

Labeling with a low eluate volume: The contents of one freeze-dried kit containing 1 mg tin(II)chloride dihydrate, 5 mg gluconic acid sodium salt and 100 μg EDTANa$_4$, dissolved in 1.25 M sodium acetate, pH 4.2, were added to 200 μg of H2BPP1 to a total volume of 100 μl. To the reaction solution, up to 1500 μl (up to 300 MBq) of a $^{188}$Re-containing generator eluate was added. The mixture was incubated at 70 °C for 60 min and the cooled at RT for 5 min. Purification of the labeled $^{188}$Re-H2BPP1 was performed using size-exclusion NAP-5 columns as described in Example 5.

Labeling with a high eluate volume: The contents of one freeze-dried kit containing 2 mg tin(II)chloride dihydrate, 50 mg gluconic acid sodium salt and 200 μg EDTANa$_4$, dissolved in 1.25 M sodium acetate, pH 4.2, were added to 200 μg of H2BPP1 to a total volume of 100 μl. To the reaction solution, 4000 μl of a $^{188}$Re-containing generator eluate was added. 440 μg ascorbic acid (2 mg/ml in 1.25 M sodium acetate buffer, pH 4.2) was added to the reaction vial and the mixture was incubated at 70 °C for 60 min and then cooled at RT for 5 min. Thereafter, the total amount of ascorbic acid in the reaction vial was adjusted to 1 mg using a solution of 5 mg/ml ascorbic acid in PBS. Purification was performed using Oasis HLB 6 cc Vac Cartridge (Waters). The cartridge was activated by passing 5 ml 50 % ethanol in water and deactivated by passing 5 ml water. The reaction mixture was passed through the cartridge, followed by 20 ml water. The cartridge was additionally washed with 500 μl 25 % ethanol in water and the purified $^{188}$Re-H2BPP1 was eluted with 1 ml 50 % ethanol in water. For further use, $^{188}$Re-H2BPP1 was diluted with water to a final ethanol concentration of 5-10 %. Purified samples were analyzed by ITLC and RP-HPLC as described in Example 5.

*Results*

Labeling with a low volume of eluate resulted in a radiochemical yield of 98.6 ± 0.5 %. The radiochemical purity of the product was over 98 %. This $^{188}$Re-H2BPP1 was used in the characterization, biodistribution and imaging studies described in Example 10-11.

Labeling with a high volume of eluate resulted in a radiochemical yield of 85 ± 10 %. The radiochemical purity of the product was over 97 %. This $^{188}$Re-H2BPP1 was used in the experimental therapy study described in Example 12.

Example 10

*In vitro* characterization of $^{188}$Re-H2BPP1 HER2-binding polypeptide

This Example describes characterization, using cell-based assays *in vitro,* of $^{188}$Re-H2BPP1 prepared as described in Example 9.

*Methods*

Cell cultures: HER2-expressing ovarian carcinoma SKOV-3 and breast carcinoma SK-BR-3 cell lines (ATCC) were used. Cells were cultured in complete medium (see Example 6) and seeded in cell culture dishes at a density of $10^6$ cells/dish. A set of three dishes was used for each data point in the *in vitro* binding specificity studies.

*In vitro* binding specificity: In control dishes, SKOV-3 and SK-BR-3 cells were pre-saturated by incubation with a 400-fold molar excess of non-labeled H2BPP1 (200 nM) for 15 min. Thereafter, 0.5 nM of the labeled polypeptide $^{188}$Re-H2BPP1 were added to both test and control dishes, and the cells were incubated in a humidified incubator (5 % $CO_2$, 37 °C) for 1 h. The medium was discarded and the cells were washed with cold, serum-free medium, before trypsin-EDTA solution (0.5 ml per dish) was added and cells incubated for an additional 10 min. Detached cells were diluted with 0.5 ml complete medium, re-suspended and transferred to fraction tubes. The radioactivity of cells was measured using an automated gamma counter with an NaI(TI) detector (1480 Wizard, Wallac) and the cell-bound radioactivity was calculated.

*In vitro* binding affinity: The binding kinetics of $^{188}$Re-H2BPP1 to HER2 receptors were measured using a LigandTracer Yellow instrument (Ridgeview Instruments) as described in Example 6.

*Results*

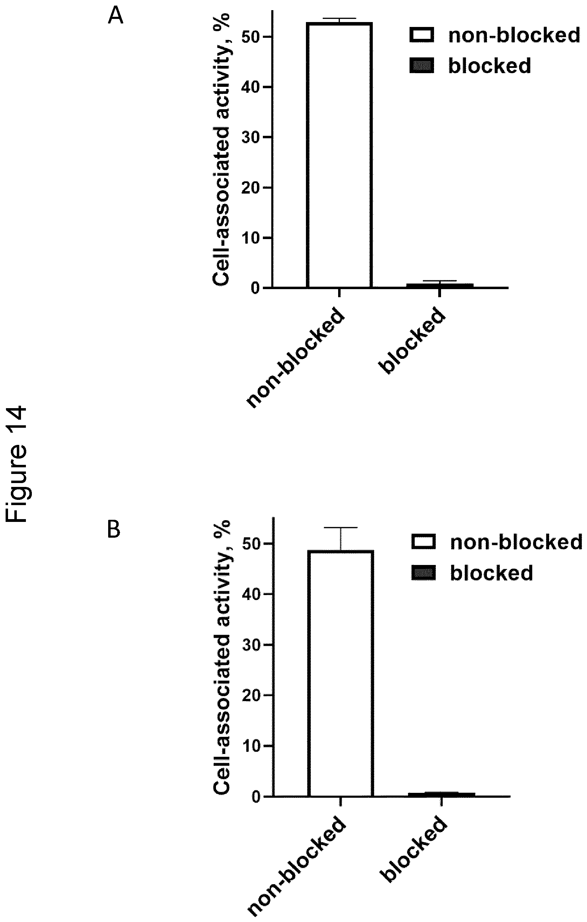

*In vitro* binding specificity: HER2-binding specificity of $^{188}$Re-H2BPP1 was evaluated using a saturation experiment. The binding was significantly (p < 5 × 10^[-5]) decreased when the cells were pre-saturated with the non-labeled H2BPP1 (Figure 14), confirming that the binding of $^{188}$Re-H2BPP1 was HER2-mediated.

*In vitro* binding affinity: The binding kinetics of $^{188}$Re-H2BPP1 to HER2 receptors on SKOV-3 cells measured using a LigandTracer are summarized in Table 11. For all conjugates, the best fit of binding to SKOV-3 cells was achieved using a 1:2 model. Interaction Map calculations showed a rapid association followed by very slow dissociation for the dominant interaction of $^{188}$Re-H2BPP1, resulting in low values of dissociation constants ($K_D$) at equilibrium, i.e. a very high affinity.

*Table 11: Apparent kinetic parameters of $^{188}$Re-labeled H2BPP1 interacting with HER2-expressing SKOV-3 cells*

| Interaction | $k_a$ (M$^{-1}$s$^{-1}$) | $k_d$ (s$^{-1}$) | $K_D$ (M) | Weight (%) |
|---|---|---|---|---|
| 1 | $[5.7\pm0.4] \times 10^5$ | $[3\pm1] \times 10^{-6}$ | $[5\pm3] \times 10^{-12}$ | 76 |
| 2 | $[1.3\pm0.3] \times 10^6$ | $[5.0\pm0.5] \times 10^{-4}$ | $[5\pm4] \times 10^{-9}$ | 6 |

*Conclusion*

The novel polypeptide $^{188}$Re-H2BPP1 was shown to bind specifically to HER2 expressing cells with a predominant affinity ($K_D$) of $5\pm3$ pM, which is favorable for retention of activity in tumors.

Example 11

*In vivo* biodistribution and imaging studies

*Methods*

Animal handling: Animal experiments were performed in accordance with the national legislation for work with laboratory animals. Approval was granted by the Ethical Committee for Animal Research in Uppsala.

Biodistribution in non-tumor-bearing mice: A biodistribution study was performed to determine if radioperrhenate is released during renal catabolism of $^{188}$Re-H2BPP1 and to obtain data for estimation of dosimetry in humans (Example 13). NMRI mice were randomized into groups of four. The average animal weight was 29.6 ± 3.4 g at the time of the experiment. Each mouse was injected intravenously with 5 μg (210 kBq) $^{188}$Re-H2BPP1 in 100 μl 0.9 % sterile saline. In a first experiment, one group of mice received 5 % sodium iodide (NaI) in drinking water 24 h before the experiment to saturate Na/I symporter in salivary gland, while another group received non-supplemented drinking water. The biodistribution was measured 4 h after injection. In further experiments, all groups received NaI in the drinking water 24 h before the experiment. The biodistribution of $^{188}$Re-H2BPP1 was measured as described in Example 7 at timepoints 0.5, 1, 4, 24 and 48 h after injection. The results obtained at 4 h after injection were compared with the results obtained in Example 7 for $^{99m}$Tc-H2BPP1 at that same timepoint to evaluate any effect of the label on the biodistribution.

Biodistribution in tumor-bearing mice: Biodistribution and targeting properties were evaluated in BALB/C nu/nu mice bearing HER2-positive SKOV-3 xenografts. To establish xenografts, $10^7$ SKOV-3 cells were subcutaneously injected into the right hind leg of female BALB/c nu/nu mice. As a specificity control, HER2-negative Ramos xenografts were used. $5\times10^6$ Ramos cells (ATCC) were subcutaneously implanted on the left hind leg of female BALB/c nu/nu in 5 mice. The experiments were performed two weeks after cell implantation. The average animal weight was 18.0±1.0 g. The average tumor weight was 0.160±0.120 and 0.7±0.3 g for SKOV-3 and Ramos xenografts, respectively. The biodistribution of $^{188}$Re-H2BPP1 was measured 1, 4, 8 and 24 h after injection in mice bearing SKOV-3 xenografts. Three groups of tumor-bearing mice were injected with 10 μg of $^{188}$Re - H2BPP1 (263 kBq, 100 μl in PBS) into the tail vein. For measurement of biodistribution at 24 and 48 h after injection, two groups of mice were injected with 470 kBq, but the injected protein mass was the same, 10 μg. To evaluate the HER2-specificity of $^{188}$Re-H2BPP1 accumulation in tumors, one group of animals bearing HER2-negative Ramos xenografts (average mouse weight was 19.8±0.6 g) was injected with 10 μg of $^{188}$Re-H2BPP1 (265 kBq, 100 μl in PBS), and the biodistribution was measured 4 h after injection. The measurement of biodistribution in tumor-bearing mice was performed as described above for NMRI mice.

*In vivo* imaging: To confirm the biodistribution results, a small animal SPECT/CT imaging was performed. One SKOV-3 xenograft mouse and one Ramos xenograft mouse were intravenously injected with 2.4 MBq/10 μg of $^{188}$Re-H2BPP1. The mouse with the SKOV-3 xenograft was imaged 1 and 4 h after injection under sevofluran anesthesia using a nanoScan SPECT/CT scanner (Mediso Medical Imaging Systems). Thereafter, mice with SKOV-3 and Ramos xenografts, respectively, were euthanized by $CO_2$ asphyxiation immediately before being placed in the camera and imaged simultaneously, side by side. The computed tomography (CT) acquisition was carried out at the following parameters: energy peak of 50 kV, 670 μA, 480 projections, 2.29 min acquisition time. SPECT acquisition was performed at the following parameters: $^{188}$Re energy peak of 150 keV, window width of 20 %, matrix of 256x256, acquisition time of 20 min. CT images were reconstructed in real-time using Nucline 2.03 Software (Mediso Medical Imaging Systems). Raw SPECT data were reconstructed using Tera-Tomo™ 3D SPECT reconstruction technology.

*Results*

Biodistribution in non-tumor-bearing mice: The results of the first experiment, evaluating blocking of Na/I symporter in NMRI mice, are presented in Figure 15A. The blocking resulted in significant (p < 0.0013) reduction of activity uptake in salivary gland. Because free radioperrhenate ([188]$ReO_4^-$) is taken up in salivary glands by the Na/I symporter, the blocking of the symporter by cold iodide shows that there is a release of radioperrhenate during catabolism of $^{188}$Re-H2BPP1. Free radioperrhenate may add to the dose absorbed by normal tissues. To avoid this, the Na/I symporter is blocked in clinics by giving patients iodine in the form of sodium iodide, iodine in milk or Lugol's iodine during radionuclide therapy. This approach can be used also for therapy with $^{188}$Re-H2BPP1. Therefore, further experiments were performed in mice after supplementation of drinking water with NaI.

Technetium and rhenium are chemical analogues, but there are differences in their chemical properties and, consequently, radiopharmaceuticals labeled with $^{99m}$Tc and $^{188}$Re exhibit different biological behavior. To ensure that such differences do not cause undesirable effects on the biodistribution of $^{188}$Re-H2BPP1 and $^{99m}$Tc-H2BPP1, the results obtained in NMRI mice 4 h after injection were compared (Figure 15B). It was found that uptake of $^{188}$Re-H2BPP1 was higher in blood, lung, liver and bone. Unexpectedly, the retention of $^{188}$Re in kidney was lower than that of $^{99m}$Tc, which should be translated into lower absorbed dose in kidneys. Since the kidney is a critical organ in radionuclide therapy, this feature of $^{188}$Re-H2BPP1 is favorable for therapeutic application of H2BPP1. Data from the biodistribution study of $^{188}$Re-H2BPP1 in normal mice are presented in Table 12. A characteristic feature of the biodistribution was a rapid clearance of $^{188}$Re-H2BPP1 from blood and other organs and tissues. Low accumulation in intestinal content suggests that the hepatobiliary excretion pathway did not play any substantial role in elimination of $^{188}$Re-H2BPP1 from the body. Very essential was the rapid reduction of the renal uptake of $^{188}$Re-H2BPP1.

*Table 12 Biodistribution of $^{188}$Re-H2BPP1 in normal NMRI mice. The data are presented as average (n=4) values ± SD.*

| Organ | Uptake, %ID/g | | | | |
|---|---|---|---|---|---|
| | **0.5 h** | **1 h** | **4 h** | **24 h** | **48 h** |
| Blood | 3.8±0.3 | 1.8±0.2 | 0.5±0.1 | 0.01±0.00 | 0.009±0.004 |
| Heart | 1.9±0.2 | 0.84±0.05 | 0.18±0.05 | 0.01±0.01 | N/A |
| Salivary gland | 1.9±0.2 | 0.9±0.1 | 0.34±0.09 | 0.01±0.00 | N/A |
| Lung | 4.5±0.7 | 2.1±0.3 | 0.6±0.2 | 0.05±0.02 | N/A |
| Liver | 3.6±0.5 | 2.4±0.3 | 1.0±0.3 | 0.20±0.04 | 0.15±0.03 |
| Spleen | 2.1±0.1 | 1.3±0.3 | 0.7±0.3 | 0.2±0.1 | 0.080±0.004 |
| Pancreas | 1.2±0.2 | 0.5±0.1 | 0.19±0.03 | 0.010±0.004 | N/A |
| Kidney | 57±7 | 29±9 | 3.1±0.8 | 0.5±0.1 | 0.17±0.02 |
| Muscle | 0.9±0.1 | 0.5±0.2 | 0.09±0.01 | 0.005±0.001 | 0.004±0.001 |
| Bone | 2.1±0.4 | 1.3±0.2 | 0.4±0.1 | 0.17±0.06 | 0.106±0.006 |
| Brain | 0.15±0.02 | 0.07±0.02 | 0.03±0.01 | 0.003±0.001 | N/A |
| Stomach* | 1.6±0.2 | 1.7±0.4 | 1.9±0.3 | 0.04±0.02 | 0.04±0.03 |
| Small intestine* | 2.2±0.5 | 1.8±0.4 | 0.5±0.2 | 0.05±0.02 | 0.04±0.04 |
| Large intestine* | 0.7±0.1 | 0.3±0.1 | 0.5±0.5 | 0.07±0.04 | 0.1±0.1 |
| Caecum* | 0.37±0.05 | 0.21±0.03 | 0.7±0.2 | 0.10±0.06 | 0.1±0.1 |

(continued)

| Organ | Uptake, %ID/g | | | | |
|---|---|---|---|---|---|
| | 0.5 h | 1 h | 4 h | 24 h | 48 h |
| Carcass** | 26±2 | 14±1 | 3.4±0.7 | 0.4±0.1 | 0.26±0.02 |
| * Data presented as %ID per whole sample with content<br>** Data presented for whole sample. | | | | | |

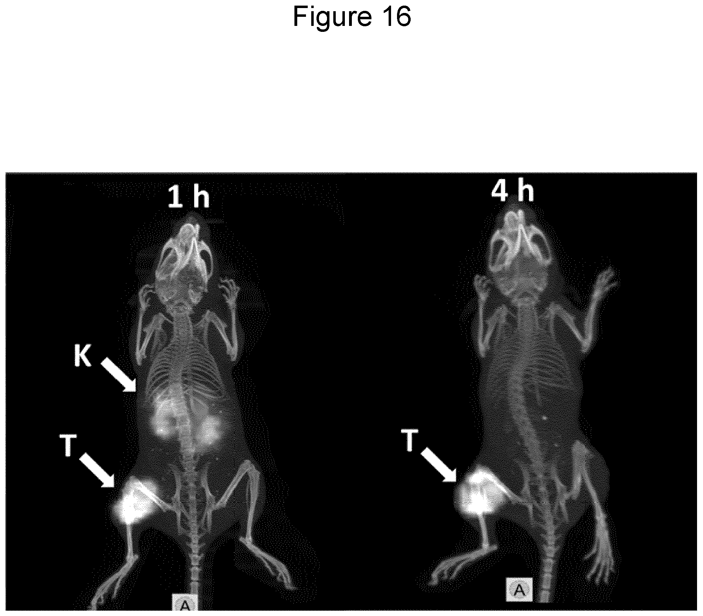

Biodistribution in tumor-bearing mice: Data from the biodistribution study of $^{188}$Re-H2BPP1 in BALB/C nu/nu mice bearing HER2-expressing SKOV-3 xenografts are presented in Figures 16-17 and Table 13. In agreement with the biodistribution data in normal NMRI mice, $^{188}$Re-H2BPP1 cleared rapidly from blood and normal tissues, including kidneys. The uptake in tumor was high, 31±4 %ID/g already 1 h after injection (Table 13). By this time point, the tumor uptake was higher than the uptake in the majority of organs and similar to the uptake in kidney. However, the tumor uptake remained high over time while the activity in kidneys declined rapidly. Already 4 h after injection, the tumor uptake was more than 8-fold higher than the renal uptake. This created a favorable precondition for radionuclide therapy. Experimental imaging using microSPECT/CT confirmed the biodistribution data (Figure 16).

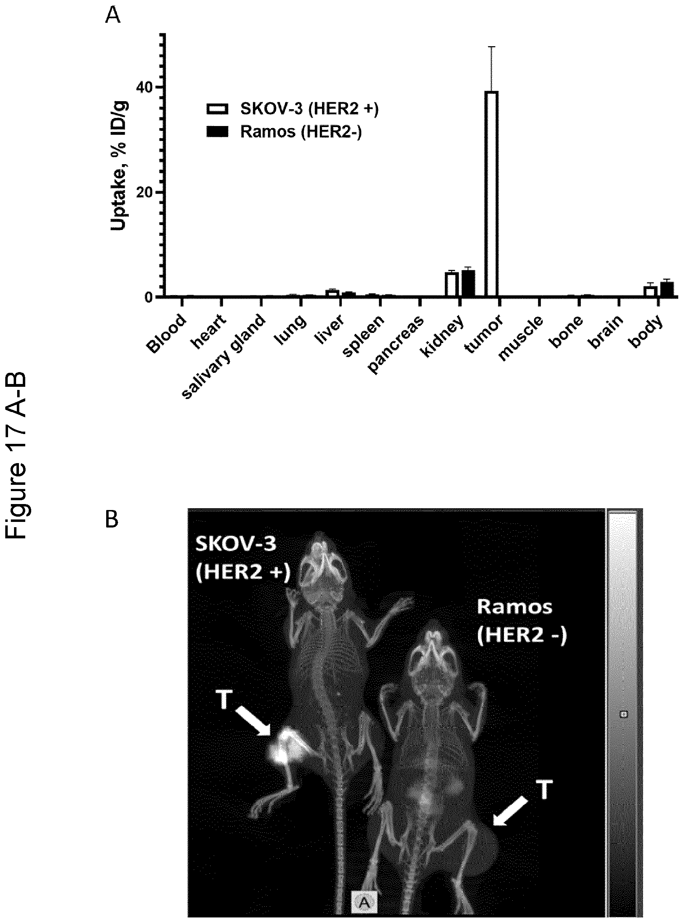

To confirm HER2-specificity of tumor targeting using $^{188}$Re-H2BPP1, its uptake was compared in HER2-positive SKOV-3 and HER2-negative Ramos lymphoma xenografts 4 h after injection (Figure 17). The uptake in the HER2-positive xenografts was 245-fold higher than in the HER2 negative xenografts. This clearly demonstrates that the tumor uptake of $^{188}$Re-H2BPP1 is HER2-mediated.

Table 13. Biodistribution of $^{188}$Re-H2BPP1 in BALB/C nu/nu mice bearing HER2-expressing SKOV-3 xenografts. The data are presented as average (n=4) values ± SD.

| Organ | Uptake, %ID/g | | | | |
|---|---|---|---|---|---|
| | 1h | 4 h | 8h | 24 h | 48 h |
| Blood | 1.8±0.5 | 0.23±0.07 | 0.036±0.003 | 0.023±0.009 | 0.005±0.005 |
| Heart | 0.9±0.1 | 0.12±0.01 | 0.03±0.01 | 0.024±0.008 | 0.02±0.02 |
| Salivary gland | 1.3±0.3 | 0.4±0.4 | 0.04±0.01 | 0.025±0.007 | 0.03±0.01 |
| Lung | 3.0±0.6 | 0.5±0.1 | 0.13±0.02 | 0.079±0.007 | 0.07±0.03 |
| Liver | 3.4±0.5 | 1.4±0.1 | 0.6±0.1 | 0.31±0.04 | 0.23±0.04 |
| Spleen | 1.7±0.2 | 0.6±0.1 | 0.23±0.03 | 0.17±0.03 | 0.12±0.01 |
| Pancreas | 0.7±0.3 | 0.11±0.04 | 0.02±0.01 | 0.017±0.001 | 0.015±0.006 |
| Kidney | 38±2 | 4.8±0.4 | 2.4±0.4 | 1.1±0.2 | 0.5±0.1 |
| Tumor | 31±4 | 39±8 | 29±6 | 12±1 | 3.8±0.6 |
| Muscle | 0.5±0.1 | 0.06±0.02 | 0.02±0.01 | 0.016±0.008 | 0.006±0.002 |
| bone | 1.6±0.4 | 0.34±0.04 | 0.20±0.04 | 0.18±0.03 | 0.18±0.06 |
| brain | 0.07±0.02 | 0.015±0.004 | 0.01±0.01 | 0.003±0.001 | 0.004 |
| Stomach* | 0.8±0.2 | 0.3±0.2 | 0.02±0.01 | 0.05±0.04 | 0.0 |
| Small intestine* | 1.2±0.3 | 0.3±0.2 | 0.07±0.01 | 0.034±0.005 | 0.018±0.007 |
| Large intestine* | 0.3±0.1 | 0.26±0.06 | 0.09±0.04 | 0.2±0.2 | 0.04±0.02 |
| Caecum* | 0.2±0.1 | 0.6±0.1 | 0.19±0.05 | 0.3±0.3 | 0.06±0.04 |
| Carcass** | 12±2 | 2.2±0.6 | 0.60±0.02 | 0.36±0.02 | 0.25±0.04 |
| * Data presented as %ID per whole sample with content<br>** Data presented for whole sample. | | | | | |

Example 12

Radiotherapy of HER2-positive xenografts

*Methods*

Animal handling: Animal experiments were performed in accordance with the national legislation for work with laboratory animals. Approval was granted by the Ethical Committee for Animal Research in Uppsala.

Experimental radionuclide therapy of xenografts: The effect of H2BPP1 on xenografts with a high level of HER2 expression was studied using SKOV-3 cells. BALB/c nu/nu mice were implanted with $10^7$ SKOV-3 cells subcutaneously on the abdomen. Seven days later, three groups of mice (n=10 per group) were treated as follows: Group A: $^{188}$Re-labeled H2BPP1 (5 $\mu$g, 16 MBq, dissolved in 150 $\mu$l 10% ethanol in water); Group B: vehicle (150 $\mu$l 10% ethanol in water; control group); and Group C: non-labelled H2BPP1 (5 $\mu$g, dissolved in 150 $\mu$l 10% ethanol in water; control group to evaluate if treatment with the protein itself has any effect on tumor growth). Three administrations, 48 h apart, were performed for each group. In line with the ethical permit, the study proceeded for 90 days after the first drug administration. The presence and size of tumors were assessed twice a week using a sliding caliper. Mice were euthanized if body weight loss exceeded 10% during one week or 15% after start of treatment, or if tumors became larger than 1 ml or ulcerated. At the treatment start, the tumor volumes were: Group A: 101 $\pm$ 28 mm$^3$; Group B: 85 $\pm$ 43 mm$^3$; Group C: 86 $\pm$ 25 mm$^3$. The difference in tumor size between the groups was not significant (p >0.05, one-way ANOVA with Bonferroni correction for multiple comparison).

*Results*

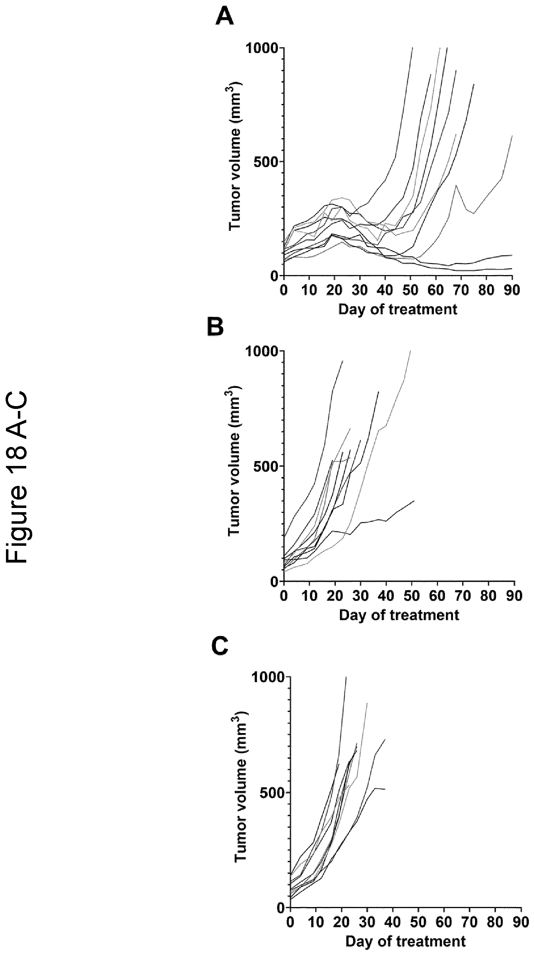

The results of the experimental radionuclide therapy of xenografts are presented in Figure 18-19. Figure 18 shows the growth of individual tumors in each group. The growth of tumors in the control groups was rapid. The tumor volume doubling time was 12 $\pm$ 7 days in Group B (treated with vehicle) and 9 $\pm$ 2 days in Group C (treated with non-labeled H2BPP1). There was no significant difference (p>0.05, unpaired t-test) in tumor doubling time between these two groups, which suggests that the treatment with non-labeled H2BPP1 has no antitumor effect. All animals in Group B were sacrificed by day 51, either due reaching the tumor volume limit or due to tumor ulceration. In group C, the last animal was sacrificed by day 37. The pattern of tumor growth in the Group A was different. A reduction of the tumor volume was observed from day 19 after treatment start (Figure 18A). At this timepoint, the mean tumor volume was significantly smaller (p <0.05, one-way ANOVA with Bonferroni correction for multiple comparison) than the tumor volume in the two control groups. After some delay, some tumors resumed growth. However, three animals were alive at the study termination (day 90).

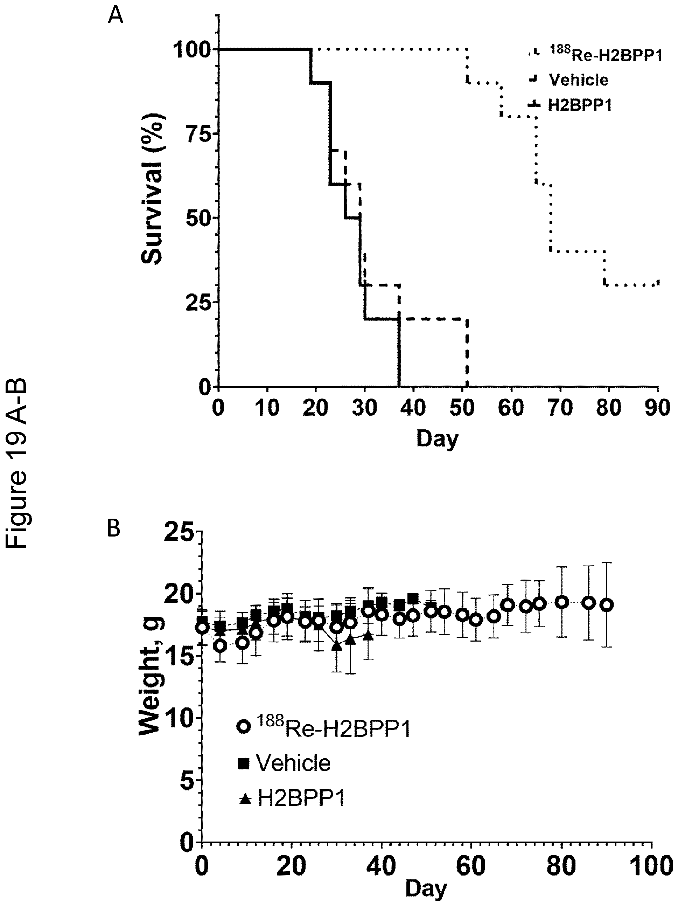

The median survival in the treatment Group (A) was 68 days, which was significantly longer (<0.0001 in the log-rank Mantel-Cox test) than in the control groups (Group B: 29 days; Group C: 27.5 days; Figure 19A). There was no difference in survival of mice treated with vehicle (Group B) and non-labeled H2BPP1 (Group C), respectively (p >0.05 in the log-rank Mantel-Cox test).

The therapy was well tolerated. The behavior and appearance of mice in the treatment group did not differ from the behavior and appearance of animals in control groups. The average animal weight did not differ significantly between the treated group and the control groups (Figure 19B).

*Conclusion*

The experimental preclinical therapy demonstrated that treatment with $^{188}$Re-H2BPP1 delays the growth of HER2-expressing tumor xenografts, significantly extends survival of mice bearing such xenografts and is not associated with adverse effects on animals.

Example 13

Dosimetry evaluation for human use

*Methods*

Upscaling of dosimetry of $^{188}$Re-H2BPP1 to human patients based on biodistribution data in non-tumor bearing mice (Example 11) was performed as described in Example 8.

*Results*

Estimated absorbed doses of the novel polypeptide $^{188}$Re-H2BPP1 in humans are given in Table 14. According to calculations using OLINDA/EXM 1.0, the effective dose should be 0.0683 mSv/MBq (Effective Dose equivalent: 0.0683 mSv/MBq).

*Table 14. Calculated absorbed dose (mGy/MBq) for $^{188}$Re-H2BPP1 in humans using OLINDA/EXM 1.0*

| Organ | Absorbed dose (mGy/MBq) | Organ | Absorbed dose (mGy/MBq) |
|---|---|---|---|
| Adrenals | 0.007 | Muscle | 0.005 |
| Brain | 0.001 | Ovaries | 0.007 |
| Breasts | 0.007 | Pancreas | 0.010 |
| Gallbladder wall | 0.007 | Red marrow | 0.012 |
| Lower large intestine wall | 0.227 | Osteogenic cells | 0.062 |
| Small intestine | 0.044 | Skin | 0.006 |
| Stomach wall | 0.208 | Spleen | 0.020 |
| Upper large intestine wall | 0.007 | Thymus | 0.007 |
| Heart wall | 0.131 | Thyroid | 0.007 |
| Kidneys | 0.244 | Urinary bladder wall | 0.007 |
| Liver | 0.029 | Uterus | 0.007 |
| Lungs | 0.031 | Total body | 0.015 |
| Effective Dose Equivalent | 0.0612 (mSv/MBq) | | |
| Effective Dose | 0.0683 (mSv/MBq) | | |

**Claims**

1. A HER2-binding polypeptide, whose amino acid sequence comprises AEAKYAKEMR NAYWEIALLP NLTNQQKRAF IRKLYDDPSQ SSELLSEAKK LSESQGGGC (SEQ ID NO:1).

2. The HER2-binding polypeptide according to claim 1, whose amino acid sequence consists of SEQ ID NO:1.

3. A radiolabeled polypeptide consisting of a radiochelate of the HER2-binding polypeptide according to any one of claims 1-2 and a radionuclide.

4. The radiolabeled polypeptide according to claim 3, wherein said radionuclide is suitable for medical imaging, for example selected from the group consisting of $^{99m}$Tc, $^{51}$Mn, $^{52m}$Mn, $^{52}$Mn, $^{186}$Re and $^{188}$Re, for example [99m]Tc.

5. The radiolabeled polypeptide according to claim 3, wherein said radionuclide is suitable for therapy, for example selected from the group consisting of $^{186}$Re and $^{188}$Re, for example [188]Re.

6. Composition comprising a HER2-binding polypeptide according to any one of claims 1-2 or a radiolabeled polypeptide according to any one of claims 3-5, and at least one pharmaceutically acceptable excipient or carrier.

7. The HER2-binding polypeptide according to any one of claims 1-2 for use as a medicament, as a diagnostic agent *in vivo* or as a prognostic agent *in vivo.*

8. The radiolabeled polypeptide according to any one of claims 3-4 for use as a diagnostic agent *in vivo.*

9. The radiolabeled polypeptide for use according to claim 8, wherein said use is in a method of diagnosing a cancer **characterized by** overexpression of HER2, comprising

i) performing a method of *in vivo* imaging of the body of a mammalian, including human, subject having or suspected of having a cancer **characterized by** overexpression of HER2, comprising the steps:

- administering a radiolabeled polypeptide according to any one of claims 3-4, optionally comprised in a composition according to claim 6, into the body of the mammalian subject, and
- obtaining one or more images of at least a part of the subject's body using a medical imaging instrument, said image(s) indicating the presence of the radionuclide inside the body; and

ii) establishing said diagnosis on the basis of the obtained images.

10. The radiolabeled polypeptide according to any one of claims 3-4 for use as a prognostic agent *in vivo.*

11. The radiolabeled polypeptide for use according to claim 10, wherein said use is in a method of establishing a prognosis for a cancer **characterized by** overexpression of HER2, comprising

i) performing a method of *in vivo* imaging of the body of a mammalian, including human, subject having or suspected of having a cancer **characterized by** overexpression of HER2, comprising the steps:

- administering a radiolabeled polypeptide according to any one of claims 3-4, optionally comprised in a composition according to claim 6, into the body of the mammalian subject, and
- obtaining one or more images of at least a part of the subject's body using a medical imaging instrument, said image(s) indicating the presence of the radionuclide inside the body; and

ii) establishing said prognosis on the basis of the obtained images.

12. The radiolabeled polypeptide according to any one of claims 3 and 5 for use as a medicament.

13. The radiolabeled polypeptide for use according to claim 12, wherein said use is in a method of treatment of a mammalian, including human, subject having a cancer **characterized by** overexpression of HER2, comprising the step of administering a therapeutically effective amount of a radiolabeled polypeptide according to any one of claims 3 and 5, optionally comprised in a composition according to claim 6, to said subject.

14. A nucleic acid encoding the polypeptide according to any one of claims 1-2.

**Patentansprüche**

1. HER2-bindendes Polypeptid, dessen Aminosäuresequenz AEAKYAKEMR NAYWEIALLP NLTNQQKRAF IRKLYDDPSQ SSELLSEAKK LSESQGGGC (SEQ ID NO: 1) umfasst.

2. HER2-bindendes Polypeptid nach Anspruch 1, dessen Aminosäuresequenz aus SEQ ID NO: 1 besteht.

3. Radioaktiv markiertes Polypeptid, bestehend aus einem Radiochelat des HER2-bindenden Polypeptids nach einem der Ansprüche 1 bis 2 und einem Radionuklid.

4. Radioaktiv markiertes Polypeptid nach Anspruch 3, wobei das Radionuklid für die medizinische Bildgebung geeignet ist, zum Beispiel ausgewählt aus der Gruppe bestehend aus $^{99m}$Tc, $^{51}$Mn, $^{52m}$Mn, $^{52}$Mn, $^{186}$Re und $^{188}$Re, zum Beispiel $^{99m}$Tc.

5. Radioaktiv markiertes Polypeptid nach Anspruch 3, wobei das Radionuklid für die Therapie geeignet ist, zum Beispiel ausgewählt aus der Gruppe bestehend aus $^{186}$Re und $^{188}$Re, zum Beispiel $^{188}$Re.

6. Zusammensetzung, umfassend ein HER2-bindendes Polypeptid nach einem der Ansprüche 1 bis 2 oder ein radioaktiv markiertes Polypeptid nach einem der Ansprüche 3 bis 5 und mindestens einen pharmazeutisch akzeptablen Hilfsstoff oder Träger.

7. HER2-bindendes Polypeptid nach einem der Ansprüche 1 bis 2 zur Verwendung als Medikament, als diagnostisches Mittel *in vivo* oder als prognostisches Mittel *in vivo.*

8. Radioaktiv markiertes Polypeptid nach einem der Ansprüche 3 bis 4 zur Verwendung als diagnostisches Mittel *in vivo*.

9. Radioaktiv markiertes Polypeptid zur Verwendung nach Anspruch 8, wobei die Verwendung in einem Verfahren zur Diagnose eines durch Überexpression von HER2 gekennzeichneten Krebses erfolgt, umfassend

   i) Durchführen eines Verfahrens zur In-vivo-Bildgebung des Körpers eines Säugetier-, einschließlich menschlichen, Subjekts, das einen durch Überexpression von HER2 gekennzeichneten Krebs hat oder bei dem dies vermutet wird, umfassend die folgenden Schritte:

   - Verabreichen eines radioaktiv markierten Polypeptids nach einem der Ansprüche 3 bis 4, das gegebenenfalls in einer Zusammensetzung nach Anspruch 6 enthalten ist, in den Körper des Säugetier-Subjekts, und
   - Erhalten eines oder mehrerer Bilder von mindestens einem Teil des Körpers des Subjekts unter Verwendung eines medizinischen Bildgebungsinstruments, wobei das (die) Bild(er) das Vorhandensein des Radionuklids im Körper anzeigt (anzeigen); und

   ii) Erstellen der Diagnose auf der Grundlage der erhaltenen Bilder.

10. Radioaktiv markiertes Polypeptid nach einem der Ansprüche 3 bis 4 zur Verwendung als prognostisches Mittel *in vivo*.

11. Radioaktiv markiertes Polypeptid zur Verwendung nach Anspruch 10, wobei die Verwendung in einem Verfahren zur Erstellung einer Prognose für einen durch Überexpression von HER2 gekennzeichneten Krebs erfolgt, umfassend

   i) Durchführen eines Verfahrens zur In-vivo-Bildgebung des Körpers eines Säugetier-, einschließlich menschlichen, Subjekts, das einen durch Überexpression von HER2 gekennzeichneten Krebs hat oder bei dem dies vermutet wird, umfassend die folgenden Schritte:

   - Verabreichen eines radioaktiv markierten Polypeptids nach einem der Ansprüche 3 bis 4, das gegebenenfalls in einer Zusammensetzung nach Anspruch 6 enthalten ist, in den Körper des Säugetier-Subjekts, und
   - Erhalten eines oder mehrerer Bilder von mindestens einem Teil des Körpers des Subjekts unter Verwendung eines medizinischen Bildgebungsinstruments, wobei das (die) Bild(er) das Vorhandensein des Radionuklids im Körper anzeigt (anzeigen); und

   ii) Erstellen der Prognose auf der Grundlage der erhaltenen Bilder.

12. Radioaktiv markiertes Polypeptid nach einem der Ansprüche 3 und 5 zur Verwendung als Medikament.

13. Radioaktiv markiertes Polypeptid zur Verwendung nach Anspruch 12, wobei die Verwendung in einem Verfahren zur Behandlung eines Säugetier-, einschließlich menschlichen, Subjekts mit einem durch Überexpression von HER2 gekennzeichneten Krebs erfolgt, umfassend den Schritt des Verabreichens einer therapeutisch wirksamen Menge eines radioaktiv markierten Polypeptids nach einem der Ansprüche 3 und 5, das gegebenenfalls in einer Zusammensetzung nach Anspruch 6 enthalten ist, an das Subjekt.

14. Nukleinsäure, die für das Polypeptid nach einem der Ansprüche 1 bis 2 kodiert.

**Revendications**

1. Polypeptide se liant à HER2, dont la séquence d'acides aminés comprend AEAKYAKEMR NAYWEIALLP NLTNQQKRAF IRKLYDDPSQ SSELLSEAKK LSESQGGGC (SEQ ID NO: 1).

2. Polypeptide se liant à HER2 selon la revendication 1, dont la séquence d'acides aminés est constituée de SEQ ID n° 1.

3. Polypeptide radiomarqué constitué d'un radiochélate du polypeptide se liant à HER2 selon l'une quelconque des revendications 1 à 2 et d'un radionucléide.

4. Polypeptide radiomarqué selon la revendication 3, dans lequel ledit radionucléide est approprié pour l'imagerie médicale, par exemple choisi dans le groupe constitué par $^{99m}Tc$, $^{51}Mn$, $^{52m}Mn$, $^{186}Re$ et $^{188}Re$, par exemple $^{99m}Tc$.

**5.** Polypeptide radiomarqué selon la revendication 3, dans lequel ledit radionucléide est approprié pour une thérapie, par exemple choisi dans le groupe constitué par $^{186}$Re et $^{188}$Re, par exemple $^{188}$Re.

**6.** Composition comprenant un polypeptide se liant à HER2 selon l'une quelconque des revendications 1 à 2 ou polypeptide radiomarqué selon l'une quelconque des revendications 3 à 5 et au moins un excipient ou véhicule pharmaceutiquement acceptable.

**7.** Polypeptide se liant à HER2 selon l'une quelconque des revendications 1 à 2 destiné à être utilisé comme médicament, comme agent de diagnostic *in vivo* ou comme agent de pronostic *in vivo*.

**8.** Polypeptide radiomarqué selon l'une quelconque des revendications 3 à 4 destiné à être utilisé comme agent de diagnostic *in vivo*.

**9.** Polypeptide radiomarqué destiné à être utilisé selon la revendication 8, dans lequel ladite utilisation dans un procédé de diagnostic d'un cancer **caractérisé par** la surexpression de HER2, comprenant

i) la réalisation d'un procédé *in vivo* d'imagerie du corps d'un mammifère, y compris d'un être humain, sujet ayant ou suspecté d'avoir un cancer **caractérisé par** la surexpression de HER2, comprenant les étapes consistant à :

- administrer un polypeptide radiomarqué selon l'une quelconque des revendications 3 à 4, éventuellement compris dans une composition selon la revendication 6, dans le corps du sujet mammifère et
- obtenir une ou plusieurs images d'au moins une partie du corps du sujet en utilisant un instrument d'imagerie médicale, ladite (lesdites) image(s) indiquant la présence du radionucléide dans le corps ; et

ii) établir ledit diagnostic sur la base des images obtenues.

**10.** Polypeptide radiomarqué selon l'une quelconque des revendications 3 à 4 destiné à être utilisé comme agent de pronostic *in vivo*.

**11.** Polypeptide radiomarqué destiné à être utilisé selon la revendication 10, dans lequel ladite utilisation dans un procédé d'établissement d'un pronostic pour un cancer **caractérisé par** la surexpression de HER2, comprenant

i) la réalisation d'un procédé d'imagerie *in vivo* du corps d'un mammifère, y compris d'un être humain, sujet ayant ou suspecté d'avoir un cancer **caractérisé par** la surexpression de HER2, comprenant les étapes consistant à :

- administrer un polypeptide radiomarqué selon l'une quelconque des revendications 3 à 4, éventuellement compris dans une composition selon la revendication 6, dans le corps du sujet mammifère et
- obtenir une ou plusieurs images d'au moins une partie du corps du sujet en utilisant un instrument d'imagerie médicale, ladite (lesdites) image(s) indiquant la présence du radionucléide dans le corps ; et

ii) établir ledit pronostic sur la base des images obtenues.

**12.** Polypeptide radiomarqué selon l'une quelconque des revendications 3 à 5 et destiné à être utilisé comme médicament.

**13.** Polypeptide radiomarqué destiné à être utilisé selon la revendication 12, dans lequel ladite utilisation est dans un procédé de traitement d'un mammifère, y compris d'un être humain, sujet ayant un cancer **caractérisé par** la surexpression de HER2, comprenant l'étape d'administration d'une quantité thérapeutiquement efficace d'un polypeptide radiomarqué selon l'une quelconque des revendications 3 à 5, éventuellement compris dans une composition selon la revendication 6, audit sujet.

**14.** Acide nucléique codant pour le polypeptide selon l'une quelconque des revendications 1 à 2.

# Figure 1

| Designation | SEQUENCE | SEQ ID NO |
|---|---|---|
| H2BPP1 | AEAKYAKEMRNAYWEIALLPNLTNQQKRAFIRKLYDDPSQSSELLSEAKKLSESQGGGC | 1 |
| H2BPP2 | AENKFNKEMRNAYWEIALLPNLTNQQKRAFIRSLYDDPSQSANLLAEAKKLNDAQGGGC | 2 |
| H2BPP3 | AENKFNKEMRNAYWEIALLPNLTNQQKRAFIRSLYDDPSQSANLLAEAKKLNDAQAPKVDC | 3 |

Figure 2

Figure 3A-B

Figure 3C-D

A

Figure 4

B

Figure 5

Figure 6

Figure 7

Figure 8

Figure 9A-D

Figure 9E-H

Figure 10

EP 4 291 251 B1

Figure 11

Figure 12

Ramos
(HER2-)

SKOV-3
(HER2+)

Figure 13

EP 4 291 251 B1

Figure 14

Figure 15 A-B

EP 4 291 251 B1

# Figure 16

Figure 17 A-B

Figure 18 A-C

A

B

Figure 19 A-B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005003156 A
- WO 2009080810 A
- WO 2015028550 A
- WO 2009016043 A
- WO 2012004384 A
- WO 2014048977 A
- WO 9101743 A

### Non-patent literature cited in the description

- **ORLOVA et al.** *Cancer Res*, 2006, vol. 66, 4339-4348
- **AHLGREN et al.** *J Nucl Med*, 2009, vol. 50, 781-789
- **BAUM et al.** *J Nucl Med*, 2010, vol. 51 (6), 892-897
- **FELDWISCH et al.** *J Mol Biol*, 2010, vol. 398, 232-247
- **SÖRENSEN et al.** *Theranostics*, 2016, vol. 6 (2), 262-271
- **SÖRENSEN et al.** *J Nucl Med*, 2014, vol. 55 (5), 730-735
- **HOFSTRÖM et al.** *J Med Chem*, 2013, vol. 56, 4966-4974
- **WÅLLBERG et al.** *J Nucl Med*, 2011, vol. 52, 461-469
- **ALTAI et al.** *Amino Acids*, 2012, vol. 5, 1975-1985
- **CYR et al.** *J Labelled Comp Radiopharm*, 2007, vol. 50 (1), S9
- **LINDBERG et al.** *Tumour Biol*, 2012, vol. 33 (3), 641-651
- **CAI et al.** *Iran J Radiol*, 20 January 2020, e96419
- **OROUJENI et al.** *Amino Acids*, 2018, vol. 50, 981-994
- **SILVERMAN G.J.** *Int. Rev. Immunol.*, 1992, vol. 9 (1), 57-78
- **NYGREN P-Å et al.** *Mol Recogn*, 1988, vol. 1, 69-74
- **AHLGREN et al.** *J Nucl Med*, 2010, vol. 50, 781-789
- **AHLGREN et al.** *Bioconjugate Chem*, 2008, vol. 19, 235-243
- **VITA et al.** *Nucleic Acids Res*, 2019, vol. 47, 339-343
- **AHLGREN et al.** *Nucl Med Biol*, 2010, vol. 37, 539-546
- **WÅLLBERG ; ORLOVA.** *Cancer Biother Radiopharm*, 2008, vol. 23, 435-442
- **STABIN.** Fundamentals of Nuclear Medicine Dosimetry. Springer, 2008, 83-86
- Report of the Task Group on Reference Man, ICRP Publication. Pergamon Press, 1975, vol. 23